(19) Europäisches Patentamt
European Patent Office
Office européen des brevets

(11) EP 0 526 279 B1

# (12) FASCICULE DE BREVET EUROPEEN

(45) Date de publication et mention de la délivrance du brevet:
**04.09.1996 Bulletin 1996/36**

(51) Int. Cl.[6]: **A61K 7/06**

(21) Numéro de dépôt: **92401888.0**

(22) Date de dépôt: **02.07.1992**

## (54) Composition pour le soin des cheveux à base d'une silicone à fonction hydroxyacylamino et de polymères substantifs

Mittel zur Haarpflege enthaltend eine, mit Hydroxyacylaminomotieven modifizierte Silicon und substantive Polymeren

Composition for the care of the hair containing hydroxyacylamino modified silicone and substantive polymers

(84) Etats contractants désignés:
**AT BE CH DE DK ES FR GB GR IT LI NL PT SE**

(30) Priorité: **04.07.1991 FR 9108388**

(43) Date de publication de la demande:
**03.02.1993 Bulletin 1993/05**

(73) Titulaire: **L'OREAL**
**F-75008 Paris (FR)**

(72) Inventeurs:
- **Dubief, Claude**
**F-78150 Le Chesnay (FR)**
- **Cauwet, Danièle**
**F-75011 Paris (FR)**

(74) Mandataire: **Casalonga, Axel et al**
**BUREAU D.A. CASALONGA - JOSSE**
**Morassistrasse 8**
**80469 München (DE)**

(56) Documents cités:
**EP-A- 0 180 464**
**EP-A- 0 342 834**
**WO-A-92/08439**
**GB-A- 2 058 103**

Remarques:
Le dossier contient des informations techniques présentées postérieurement au dépôt de la demande et ne figurant pas dans le présent fascicule.

EP 0 526 279 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.13.4/3.4

## Description

La présente invention est relative à des compositions de lavage et/ou de conditionnement des matières kératiniques, notamment les cheveux, à base d'une silicone à fonction hydroxyacylamino et de polymères substantifs, ainsi qu'aux procédés de traitement de ces matières mettant en oeuvre de telles compositions.

Il est bien connu que les cheveux sont sensibilisés ou fragilisés à des degrés divers par l'action des agents atmosphériques, tels que le soleil, les intempéries, ainsi que par l'action de différents traitements cosmétiques comme les permanentes, défrisages, teintures ou décolorations. Les cheveux deviennent alors difficiles à démêler et à coiffer. Par ailleurs, ils sont rêches au toucher et manquent de douceur.

On recherche, de ce fait, des compositions de lavage et/ou de conditionnement des matières kératiniques qui pourraient conférer, notamment aux fibres telles que les cheveux, de bonnes propriétés de démêlage associées à de bonnes propriétés de douceur.

On a déjà utilisé dans le passé des compositions de shampooing contenant un polymère substantif. Ces compositions sont connues pour améliorer le démêlage des cheveux.

On connaît également des compositions de shampooing renfermant des silicones qui donnent généralement de bonnes propriétés de douceur aux cheveux.

On connait par le document GB-A 2.058.103 des compositions de conditionnement contenant des silicones, en particulier des silicones aminées et des polymères cationiques. Ces compositions n'ont aucune propriété détergente.

On connaît, par ailleurs, des compositions contenant des silicones et des polymères, notamment des polymères cationiques.

Cependant, ces compositions se sont révélées non satisfaisantes pour conférer toutes les caractéristiques de démêlage et de douceur souhaitées aux matières kératiniques traitées, notamment aux cheveux.

La demanderesse a découvert, ce qui fait l'objet de l'invention, que l'utilisation d'une silicone particulière à fonction hydroxyacylamino et d'un polymère substantif dans une base tensio-active détergente, conduisait à des propriétés de démêlage et de douceur supérieures à celles obtenues avec chacun des constituants pris séparément dans la même base et à la concentration totale de l'association.

On a constaté, par ailleurs, que cette association conduisait à des propriétés de démêlage et de douceur, notamment sur les cheveux, améliorées par rapport aux propriétés que l'on pouvait obtenir avec les associations connues dans l'état de la technique.

Ces compositions, dans leur forme de réalisation préférée, contiennent des agents de mise en suspension, différents des alcools ayant 27 à 44 atomes de carbone et comportant un ou deux groupements éther et/ou thioéther ou sulfoxyde.

L'invention a donc pour objet une composition de lavage et/ou de conditionnement des matières kératiniques renfermant au moins une silicone à fonction hydroxyacylamino, au moins un polymère substantif, au moins un agent tensio-actif détergent et éventuellement certains agents de mise en suspension des silicones.

L'invention a également pour objet un procédé de lavage et/ou de conditionnement des matières kératiniques, en particulier des cheveux, mettant en oeuvre une telle composition.

D'autres objets de l'invention apparaîtront à la lecture de la description et des exemples qui suivent.

La composition de lavage et/ou de conditionnement des matières kératiniques, conforme à l'invention, est essentiellement caractérisée par le fait qu'elle contient dans un milieu aqueux, au moins une silicone à fonction hydroxyacylamino, au moins un polymère substantif et au moins un agent tensio-actif ayant des propriétés détergentes.

La silicone à fonction hydroxyacylamino est choisie notamment parmi les composés répondant à la formule :

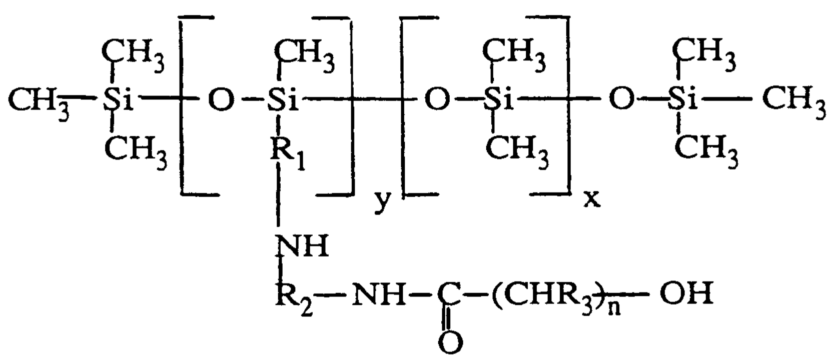

(I)

dans laquelle :

$R_1$ et $R_2$ désignent $(CH_2)_2$, $(CH_2)_3$, $(CH_2)_4$, $-CH_2CH(CH_3)CH_2-$ ;

$R_3$ désigne hydrogène, hydroxyle, alkyle, hydroxyalkyle;
n est un nombre entier variant de 2 à 7;
x est un nombre entier variant de 20 à environ 1500;
y varie entre environ 0,5 et 10;
et le rapport y/x est inférieur à 0,05.

De telles silicones sont plus particulièrement décrites dans la demande de brevet européen EP-A-O342834.

Une silicone de formule (I) plus particulièrement préférée est celle répondant à la formule (I), dans laquelle :
$R_1$ désigne $-CH_2CH(CH_3)CH_2-$;
$R_2$ désigne $-(CH_2)_2-$ ;
$R_3$ désigne hydrogène;
x est égal à 392;
y est égal à 8;
n est égal à 3.

De telles silicones sont vendues en particulier sous la dénomination Q2-8413 par la Société DOW CORNING.

Les silicones à fonction hydroxyacylamino sont présentes dans les compositions conformes à l'invention dans des proportions de préférence comprises entre 0,1 et 20% en poids, et en particulier entre 0,5 et 10% en poids par rapport au poids total de la composition.

On appelle "polymère substantif" un polymère révélable à l'aide du colorant acide Red 80 selon RICHARD J. CRAWFORD, Journal of the Society of Cosmetic Chemists, 1980 31 (5), pages 273 à 278.

Les polymères substantifs sont choisis en particulier parmi des polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000 et sont différents du copolymère de diallyl dialkyl ammonium et d'un monomère anionique tel que l'acide acrylique.

Parmi ces polymères, on peut citer plus particulièrement les protéines quaternisées, les polymères de la famille des polyamines, polyaminoamides, polyammonium quaternaires.

**A.** Les protéines quaternisées sont en particulier des polypeptides modifiés chimiquement et portant en bout de chaîne ou greffés sur celle-ci, des groupements ammonium quaternaires. Parmi ces protéines, ont peut citer notamment :

- les hydrolysats de collagène portant des groupements triéthylammonium tels que les produits vendus sous la dénomination "QUAT-PRO E" par la Société MAYBROOK et dénommés dans le dictionnaire CTFA "Triéthonium Hydrolyzed Collagen Ethosulfate";
- les hydrolysats de collagène portant des groupements chlorure de triméthylammonium ou de diméthylstéarylammonium vendus sous la dénomination de "QUAT-PRO S " par la Société MAYBROOK et dénommés dans le dictionnaire CTFA "Steartrimonium Hydrolyzed Collagen";
- les hydrolysats de protéines animales portant des groupements diméthylbenzylammonium tels que les produits vendus sous la dénomination "CROTEIN BTA" par la société CRODA et dénommés dans le dictionnaire CTFA "Benzyltrimonium Hydrolyzed Animal Protein";
- les hydrolysats de protéines portant sur la chaîne polypeptidique des groupements ammonium quaternaires comportant au moins un radical alkyle ayant de 1 à 18 atomes de carbone.

Parmi ces hydrolysats de protéines, on peut citer entre autres :

- le CROQUAT L dont la chaîne polypeptidique a un poids moléculaire moyen d'environ 2500 et dont le groupement ammonium quaternaire comporte un groupement alkyle en $C_{12}$;
- le CROQUAT M dont la chaîne polypeptidique a un poids moléculaire moyen d'environ 2500 et dont le groupement ammonium quaternaire comporte un groupement alkyle en $C_{10}$-$C_{18}$;
- le CROQUAT S dont la chaîne polypeptidique a un poids moléculaire moyen d'environ 2700 et dont le groupement ammonium quaternaire comporte un groupement alkyle en $C_{18}$;
- le CROQUAT Q dont la chaîne polypeptidique a un poids moléculaire moyen de l'ordre de 12 000 et dont le groupement ammonium quaternaire comporte au moins un groupe alkyle ayant de 1 à 18 atomes de carbone;

- une protéine quaternisée végétale de soja vendue sous la dénomination CROQUAT SOYA.

Ces différents produits sont vendus par la Société CRODA.

- une protéine quaternisée résultant de la condensation de la cocamidopropyldiméthylamine sur une protéine animale hydrolysée, dénommée, dans le supplément de la 3ème édition (1982) du dictionnaire CTFA, COCAMIDOPROPYLDIMONIUM HYDROXYPROPYLAMINO HYDROLYSED ANIMAL PROTEIN, vendue sous la dénomination LEXEIN QX 3000 par la Société INOLEX.

**B.** Les polymères de la famille des polyamines, polyaminoamides ou polyammonium quaternaires, utilisables conformément à la présente invention, sont décrits en particulier dans les brevets français de la demanderesse n° 2 505 348 ou 2 542 997.

Parmi ces polymères, on peut citer :

(1) les copolymères vinylpyrrolidone/acrylate ou méthacrylate de dialkylaminoalkyle quaternisés ou non, tels que les produits vendus sous la dénomination "GAFQUAT" par la Société GAF CORPORATION comme par exemple "GAFQUAT 734 ou 755", ou bien les produits dénommés "COPOLYMER 845, 958 et 937". Ces polymères sont décrits en détail dans les brevets français 2 077 143 et 2 393 573.

(2) Les dérivés d'éthers de cellulose comportant des groupements ammonium quaternaires décrits dans le brevet français 1 492 597 et en particulier les polymères commercialisés sous les dénominations "JR" (JR 400, JR 125, JR 30M) ou "LR" (LR 400, LR 30M) par la Société UNION CARBIDE CORPORATION. Les polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés par un monomère hydrosoluble d'ammonium quaternaire et décrits plus en détail dans le brevet américain 4 131 576 tels que les hydroxyalkylcelluloses comme les hydroxyméthyl-, hydroxyéthyl- ou hydroxypropylcellulose greffées par un sel de méthacryloyléthyltriméthylammonium, méthacrylamidopropyltriméthylammonium, diméthyldiallylammonium.

Les produits commercialisés répondant à cette définition sont plus particulièrement les produits vendus sous les dénominations "CELQUAT L 200" et "CELQUAT H 100" par la Société NATIONAL STARCH.

(4) Les polysaccharides quaternisés décrits plus particulièrement dans les brevets américains 3 589 578 et 4 031 307 et plus particulièrement le produit commercialisé sous la dénomination "JAGUAR C 13 S" vendu par la Société MEYHALL.

(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont décrits dans les brevets français 2 162 025 et 2 280 361.

(6) Les polyaminoamides solubles dans l'eau préparés en particulier par polycondensation d'un composé acide avec une polyamine. Ces polyaminoamides peuvent être réticulés par une épihalohydrine, un diépoxyde, un dianhydride, un anhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé; l'agent réticulant étant utilisé dans des proportions allant de 0,025 à 0,35 mole par groupement amine du polyaminoamide.

Ces polyaminopolyamides peuvent être alcoylés ou s'ils comportent une ou plusieurs fonctions amines tertiaires, quaternisés. De tels polymères sont décrits en particulier dans les brevets français 2 252 840 et 2 368 508.

(7) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont décrits dans le brevet français 1 583 363.

Parmi ces dérivés, on peut citer plus particulièrement les polymères acide adipique/diméthylaminohydroxypropyl/diéthylène triamine vendus sous les dénominations "CARTARETINE F, $F_4$ ou $F_8$" par la Société SANDOZ.

(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre la polyalkylène polyamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont décrits en particulier dans les brevets américains 3 227 615 et 2 961 347.

Des polymères de ce type sont en particulier commercialisés sous la dénomination "HERCOSETT 57" par la Société HERCULES INCORPORATED ou bien sous la dénomination de "PD 170" ou "DELSETTE 101" par la Société HERCULES dans le cas du copolymère d'acide adipique/époxypropyl/diéthylène-triamine.

(9) Les cyclopolymères ayant un poids moléculaire de 20 000 à 3 000 000 tels que les homopolymères comportant comme constituant principal de la chaîne des motifs répondant aux formules (II) ou (II') :

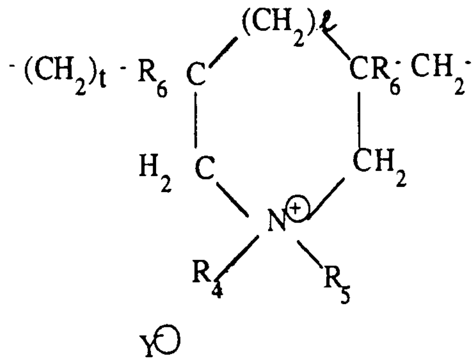

(II)

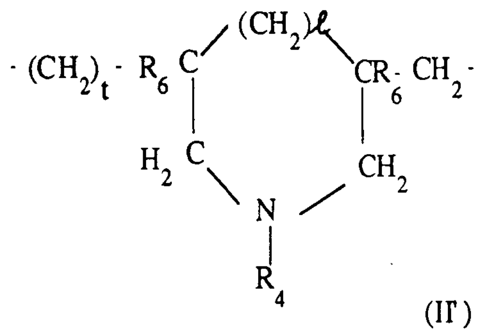

(II')

$\ell$ et t sont égaux à 0 ou 1, et le somme $\ell + t = 1$ ;

$R_6$ désigne hydrogène ou méthyle;

$R_4$ et $R_5$ désignent, indépendamment l'un de l'autre, un groupement alkyle ayant de 1 à 22 atomes de carbone, un groupement hydroxyalkyle dans lequel le groupement alkyle a de préférence 1 à 5 atomes de carbone, un groupement amidoalkyle inférieur et où $R_4$ et $R_5$ peuvent désigner conjointement avec l'atome d'azote auquel ils sont rattachés, des groupements hétérocycliques tels que pipéridinyle ou morpholinyle, ainsi que les copolymères comportant des unités de formules (II) ou (II') et des unités dérivés d'acrylamide ou de diacétone acrylamide;

$Y^{\ominus}$ est un anion tel que bromure, chlorure, acétate, borate, citrate, tartrate, bisulfate, bisulfite, sulfate, phosphate.

Parmi les polymères définis ci-dessus, on peut citer plus particulièrement l'homopolymère de chlorure de diméthyldiallylammonium vendu sous la dénomination "MERQUAT 100", ayant un poids moléculaire inférieur à 100 000, le copolymère de chlorure de diméthyldiallylammonium et d'acrylamide ayant un poids moléculaire supérieur à 500 000 et vendu sous la dénomination "MARQUAT 550" par la Société MERCK.

Ces polymères sont décrits plus particulièrement dans le brevet français 2 080 759, et son certificat d'addition n° 2 190 406.

(10) Le polymère de polyammonium quaternaire contenant des motifs récurrents répondant à la formule :

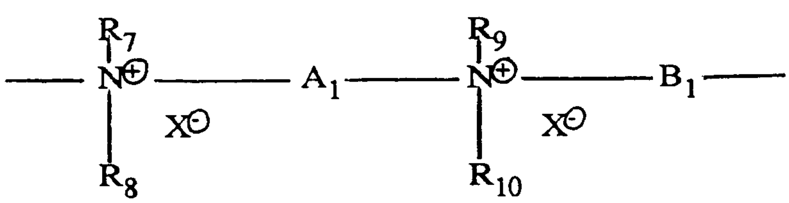

(III)

dans laquelle $R_7$ et $R_8$, $R_9$ et $R_{10}$ étant identiques ou différents, représentent des radicaux aliphatiques, alicycliques ou arylaliphatiques contenant de 1 à 20 atomes de carbone ou des radicaux hydroxy alkylaliphatiques inférieurs, ou bien $R_7$ et $R_8$ et $R_9$ et $R_{10}$ ensemble ou séparément, constituent avec les atomes d'azote auxquels ils sont rattachés, des hétérocycles contenant éventuellement un second hétéroatome autre que l'azote ou bien $R_7$, $R_8$, $R_9$ et $R_{10}$ représentent un radical alkyle en $C_1$-$C_6$ linéaire ou ramifié substitué par un groupement nitrile, ester, acyle, amide ou

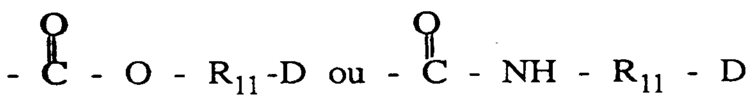

où $R_{11}$ est un alkylène et D un groupement ammonium quaternaire.

$A_1$ et $B_1$ représentent des groupements polyméthyléniques contenant de 2 à 20 atomes de carbone pouvant être linéaires ou ramifiés, saturés ou insaturés, et pouvant contenir, liés à ou intercalés dans la charrie principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et

$X^{\ominus}$ désigne un anion dérivé d'un acide minéral ou organique.

$A_1$ et $R_7$ et $R_9$ peuvent former avec les deux atomes d'azote auxquels ils sont rattachés, un cycle pipérazinique; en outre, si $A_1$ désigne un radical alkylène ou hydroxyalkylène linéaire ou ramifié, saturé ou insaturé, $B_1$ peut également désigner un groupement :

$$(CH_2)_n - CO - D - OC - (CH_2)_n -$$

dans lequel D désigne :

a) un reste de glycol de formule : - O - Z - O -
où Z désigne un radical hydrocarboné linéaire ou ramifié ou un groupement répondant aux formules :

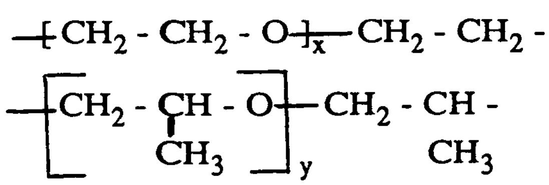

où x et y désignent un nombre entier de 1 à 4, représentant un degré de polymérisation défini et unique ou un nombre quelconque de 1 à 4 représentant un degré de polymérisation moyen;
b) un reste de diamine bis-secondaire tel qu'un dérivé de pipérazine;
c) un reste de diamine bis-primaire de formule :

$$- NH - Y - NH -$$

où Y désigne un radical hydrocarboné linéaire ou ramifié , ou bien le radical bivalent

$$- CH_2-CH_2-S-S-CH_2-CH_2-$$

d) un groupement uréylène de formule :

$$- NH - CO - NH - ;$$

$X^{\ominus}$ est un anion tel que chlorure ou bromure.

Ces polymères ont une masse moléculaire généralement comprise entre 1000 et 100 000.

Des polymères de ce type sont décrits en particulier dans les brevets français 2 320 330, 2 270 846, 2 316 271, 2 336 434, et 2 413 907 et les brevets US-A-2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.

(11) Les polymères de polyammonium quaternaires constitués de motifs de formule :

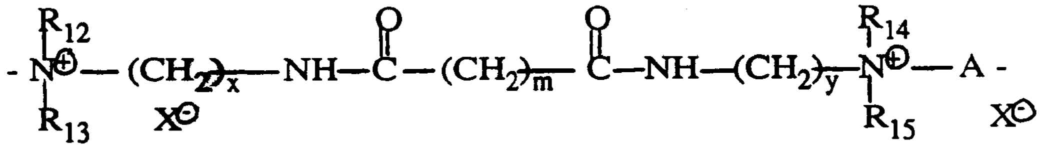 (IV)

dans laquelle :
$R_{12}$, $R_{13}$, $R_{14}$ et $R_{15}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à 0 ou un nombre entier compris entre 1 et 6, sous réserve que $R_{12}$, $R_{13}$, $R_{14}$ et $R_{15}$ ne représentent pas simultanément un atome d'hydrogène;
x et y, identiques ou différents, sont des nombres entiers compris entre 1 et 6;
m est égal à 0 ou à un nombre entier compris entre 1 et 34;
x désigne un atome d'halogène;
A désigne un radical d'un dihalogénure et représente de préférence

$$- CH_2 - CH_2 - O- CH_2 - CH_2 -$$

De tels composés sont décrits plus en détail dans la demande EP-A-122 324.

On peut par exemple citer parmi ceux-ci, les produits "MIRAPOL A 15", "MIRAPOL AD1", "MIRAPOL AZ1", "MIRAPOL 175", vendus par la Société MIRANOL.

(12) Les homopolymères ou copolymères dérivés d'esters ou d'amides acrylique ou méthacrylique et comportant les motifs :

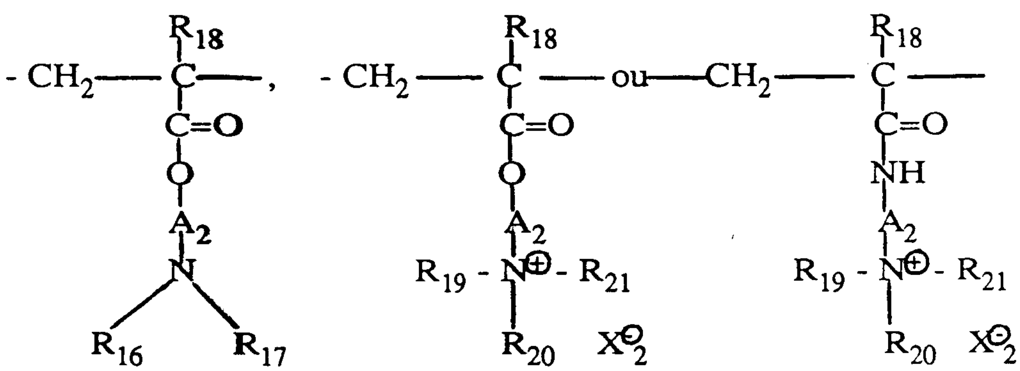 (V)

dans lesquels :
$R_{18}$ désigne H ou $CH_3$;
$A_2$ est un groupe alkyle linéaire ou ramifié de 1 à 6 atomes de carbone ou un groupe hydroxyalkyle de 1 à 4 atomes de carbone;
$R_{19}$, $R_{20}$, $R_{21}$ identiques ou différents, représentent un groupe alkyle ayant de 1 à 18 atomes de carbone ou un radical benzyle;
$R_{16}$ et $R_{17}$ représentent hydrogène ou un groupe alkyle ayant de 1 à 6 atomes de carbone;
$X^{\ominus}{}_2$ désigne un anion méthosulfate ou un halogénure tel que chlorure ou bromure.

Le ou les comonomères utilisables appartiennent à la famille des acrylamide, méthacrylamide, diacétone acrylamide, acrylamide et méthacrylamide substitués à l'azote par des alkyles inférieurs, des acides acrylique ou méthacrylique ou leurs esters, la vinylpyrrolidone, des esters vinyliques.

Parmi ces composés, on peut citer le copolymère d'acrylamide et de diméthylaminoéthyl méthacrylate quaternisé au sulfate de diméthyle et vendu sous la dénomination "HERCOFLOC" par la Société HERCULES, le copolymère d'acrylamide et de chlorure de méthacryloyloxyéthyltriméthylammonium - décrit dans la demande de brevet EP-A-80976 - et vendu sous la dénomination "BINA QAT P100" par la Société CIBA GEIGY, ou encore le poly(chlorure de méthacrylamidopropyltriméthylammonium) vendu sous la dénomination "POLYMAPTAC" par la Société TEXACO CHEMICALS, le méthacryloyloxyéthyltriméthylammonium métho-sulfate et son copolymère avec l'acrylamide vendus sous la dénomination "RETEN" par la Société HERCULES.

(13) Les polymères quaternaires de vinylpyrrolidone et de vinylimidazole tel que par exemple les produits commercialisés sous les dénominations "LUVIQUAT FC 905, FC 550 et FC 370" par la société BASF.

(14) Les polyamines comme le Polyquart H vendu par HENKEL, référencé sous le nom de "POLYETHYLENEGLYCOL (15) TALLOW POLYAMINE" dans le dictionnaire CTFA.

D'autres polymères substantifs utilisables conformément à l'invention sont des polyalkylèneimines, en particulier des polyéthylèneimines, des polymères contenant des motifs vinylpyridine ou vinylpyridinium, des condensats de polyamines et d'épichlorhydrine, les polyuréylènes quaternaires et les dérivés de la chitine.

On peut citer également les polymères dérivés du chitosane choisis en particulier parmi ceux décrits dans le brevet français 2 137 684 et comportant des motifs répondant aux formules (VI):

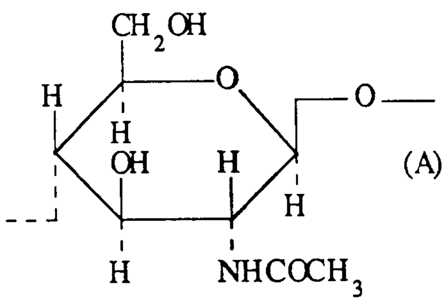
(A)

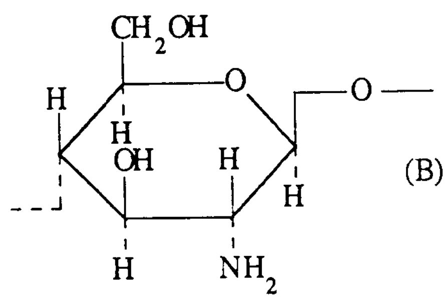
(B)

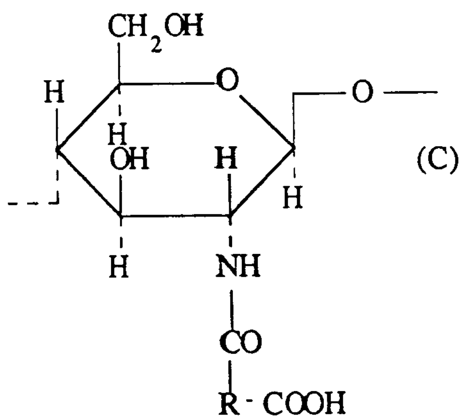
(C)

(VI)

Le motif (A) est présent dans des proportions de 0 à 30%, le motif (B) de 5 à 50%, le motif (C) de 30 à 90% en poids. R représente un groupement alkylène linéaire ou ramifié comportant de 2 à 5 atomes de carbone.

Le polymère préféré comporte de préférence 0 à 20% de motif (A), 40 à 50% de motif (B) et 40 à 50% de motif (C) dans lequel R désigne un radical alkylène et de préférence $-CH_2-CH_2-$.

Un tel polymère sera désigné dans les exemples par "polymère P1".

Les polymères particulièrement préférés dans les compositions conformes à l'invention sont les suivants :

- le copolymère bloc comportant les motifs de formule :

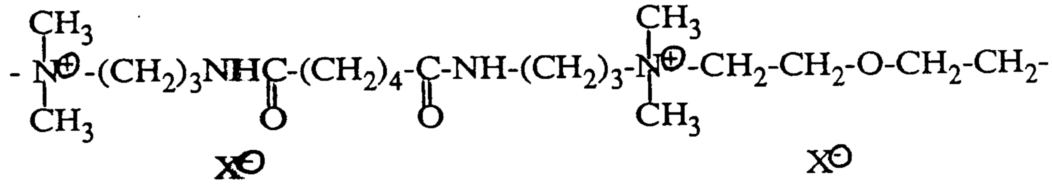

- et de formule :

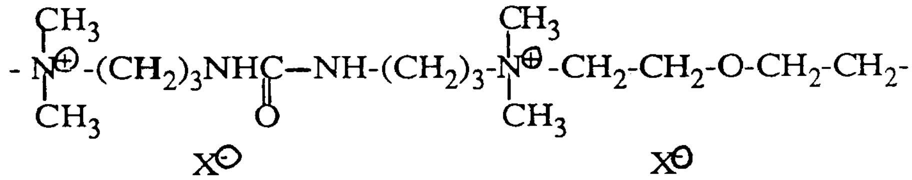

$X^{\ominus}$ est un halogénure, vendu sous la dénomination "MIROPOL 175" par la Société MIRANOL.

- les cyclopolymères de diméthyldiallylammonium vendus sous les dénominations "MERQUAT 100", "MERQUAT 550" par la Société MERCK;

- les dérivés de chitosane.

Les polymères substantifs utilisables selon l'invention sont présents dans les compositions dans des proportions comprises entre 0,05 et 8% en poids et de préférence entre 0,1 et 5% en poids par rapport au poids total de la composition.

Lorsque les compositions sont utilisées comme compositions de lavage, elles contiennent des agents tensio-actifs détergents choisis en particulier parmi les agents tensio-actifs anioniques, amphotères, zwittérioniques, non-ioniques ou leurs mélanges.

Parmi les agents tensio-actifs anioniques, on peut citer les sels alcalins, les sels d'ammonium, les sels d'amines, les sels d'aminoalcools, les sels de magnésium des composés suivants : les alkyléther sulfates, alkylamidoéthersulfates, alkylarylpolyéthersulfates, mono-glycérides sulfates; les arylsulfonates, alkylamides sulfonates, alkyl arylsulfonates, oléfines sulfonates, parafines sulfonates; les alkyl sulfosuccinates, les alkyléthersulfosuccinates, les alkylamides sulfosuccinates; les alkylsulfosuccinamates; les alkylsulfoacétates; les alkylétherphosphates; les acylsarcosinates, les N-acyltaurates.

Le radical alkyle ou acyle de ces différents composés est généralement constitué par une chaîne carbonée comportant de 12 à 20 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 20 atomes de carbone.

On peut également utiliser des agents tensio-actifs faiblement anioniques, tels que les acides éthers carboxyliques polyoxyalkylénés, en particulier ceux comportant 2 à 50 groupements oxyde d'éthylène.

Les agents tensio-actifs non-ioniques sont plus particulièrement choisis parmi les alcools ou les $\alpha$-diols ou les alkylphénols ou les acides gras polyéthoxylés, polypropoxylés ou polyglycérolés, à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'ethylène; les esters d'acides gras du sorbitan oxyéthylénés ayant de préférence 2 à 30 moles d'oxyde d'éthylène; les esters d'acides gras de sucrose, les esters d'acides gras du polyéthylène glycol, les esters d'acides gras de glycols, les alkylpolyglycosides, les oxydes d'amines tels que les oxydes d'alkyl($C_{10}$-$C_{14}$)amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl($C_8$-$C_{20}$)bétaïnes, les sulfobétaïnes, les alkyl($C_8$-$C_{20}$)amidoalkyl($C_1$-$C_6$)bétaïnes ou les alkyl($C_8$-$C_{20}$)amidoalkyl($C_1$-$C_6$)sulfobétaïnes.

Parmi les dérivés d'amines, on peut citer les produits vendus sous la dénomination "MIRANOL", tels que décrits dans les brevets US-A-2 528 378 et 2 781 354 et classés dans le dictionnaire CTFA, 3ème édition, 1982, sous la dénomination d'Amphocarboxyglycinates et Amphocarboxypropionates.

Les agents tensio-actifs sont utilisés dans les compositions conformes à l'invention dans des proportions suffisantes pour conférer un caractère détergent à la composition et sont comprises de préférence entre 5 et 50% en poids par rapport au poids total de la composition et en particulier entre 8 et 35%.

Les compositions conformes à l'invention ont généralement un pH compris entre 2 et 9 et en particulier entre 3 et 8.

Le milieu aqueux peut être constitué uniquement par de l'eau ou par un mélange d'eau et d'un solvant cosmétiquement acceptable, tel qu'un alcool inférieur en $C_1$-$C_4$ comme l'éthanol, l'isopropanol, le n-butanol, les alkylèneglycols comme l'éthylèneglycol ou les éthers de glycols.

Les compositions conformes à l'invention peuvent également contenir sous une forme de réalisation préférée, des agents de mise en suspension différents des alcools ayant 27 à 44 atomes de carbone et comportant un ou deux groupements éther et/ou thioéther ou sulfoxyde.

Parmi ces agents, on peut citer :

a) les composés de formule :

$$R_{22}X \qquad \text{(III)}$$

dans laquelle $R_{22}$ est un radical aliphatique à longue chaîne carbonée, éventuellement interrompue par des atomes d'oxygène, et X est un reste acide carboxylique, sulfurique ou phosphorique ou un radical dérivé d'un acide carboxylique ou d'un amide; ces composés de formule (III) sont choisis parmi ceux dans lesquels :

(i) $R_{22}$ est un radical alkyle ou alcényle en $C_{11}$-$C_{21}$ et X est :

. un groupement COOD où D est un radical mono- ou polyhydroxyalkyle dérivé d'un polyol en $C_2$-$C_3$ ou un radical $CH_2CH_2SO_3M$,

. un groupement $CO(OCH_2CH_2)_n$-OH où n a une valeur comprise entre 2 et 150,

. un groupement

$$COOCH_2-\underset{\displaystyle CH_3}{\underset{|}{CH}}-(OCH_2CH_2)_nOH$$

où n a une valeur comprise entre 2 et 150, les fonctions OH libres des groupements définis ci-dessus pouvant être estérifiées par un acide $R_{22}COOH$ où $R_{22}$ est un alkyle ou un alcényle en $C_{11}$-$C_{21}$,

. un groupement $CONR_{23}R_{24}$ où $R_{23}$ et $R_{24}$ représentent hydrogène ou hydroxyalkyle en $C_1$-$C_4$, l'un au moins représentant hydroxyalkyle en $C_1$-$C_4$,

. un groupement $OSO_3M$ ou 1/3 $PO_4^{3-}M_3$ où M représente un métal alcalin, ammonium ou un reste d'alcoolamine en $C_1$-$C_4$.

(ii) $R_{22}$ désigne un radical $R_{25}(OC_2H_4)_\ell OCH_2$ et X désigne un groupement COOM où M a la signification indiquée ci-dessus, $R_{25}$ désignant un radical alkyle en $C_{12}$-$C_{14}$ et $\ell$ un nombre entier ou décimal compris entre 2,5 et 10, ou bien $R_{25}$ désigne oléyle et $\ell$ varie de 2 à 9 ou encore $R_{25}$ désigne alkyle en ($C_8$-$C_9$)phényle et $\ell$ varie de 4 à 8, ou les dérivés dans lesquels $R_{22}$ désigne un groupement alkyle ($C_{12}$-$C_{16}$) éther et X un groupement $CONR_{23}R_{24}$, dans lequel $R_{23}$ et $R_{24}$ ont la même signification que celle indiquée ci-dessus;

b) des oxydes de diméthylalkyl($C_{16}$-$C_{22}$)amines;
c) les biopolysaccharides choisis par exemple parmi les gommes de xanthane et les scléroglucanes.

Ces agents de suspension sont utilisés dans les compositions conformes à l'invention dans des proportions généralement comprises entre 0,1 et 20% en poids et de préférence entre 0,5 et 10% en poids par rapport au poids total de la composition.

Les compositions conformes à l'invention peuvent également contenir des agents nacrants ou opacifiants dans des proportions pouvant aller jusqu'à 3%, tels que des palmitates de sodium ou de potassium, des stéarates ou hydroxystéarates de sodium ou de potassium, le mono- ou distéarate d'éthylèneglycol.

Ces compositions peuvent également contenir des agents régulateurs de viscosité, tels que des électrolytes comme le chlorure de sodium ou le xylènesulfonate de sodium, des hydrotropes, des épaississants comme les dérivés de cellulose, tels que par exemple la carboxyméthylcellulose, l'hydroxypropylcellulose, l'hydroxyéthylcellulose, la gomme de guar, des gommes de guar hydroxypropylées.

Ces agents régulateurs de viscosité sont utilises dans des proportions pouvant aller jusqu'à 10% en poids par rapport au poids total de la composition et de préférence inférieure à 5% en poids.

Les compositions peuvent également contenir d'autres agents ayant pour effet d'améliorer les propriétés cosmétiques des cheveux et/ou de la peau, sous réserve qu'ils n'altèrent pas la stabilité des compositions, parmi lesquels on peut citer des agents tensio-actifs cationiques, des polymères ou des protéines autres que les protéines quaternisées, des huiles, des cires, des résines ou gommes de silicone autres que les dérivés de silicone définis ci-dessus.

Les polymères, les agents tensio-actifs cationiques, les protéines non quaternisées et les silicones autres que les silicones à groupement hydroxyacylamino de formule (I), sont utilisés de préférence dans des proportions comprises entre 0,05 et 6%, et en particulier entre 0,1 et 3% en poids par rapport au poids total de la composition.

Ces compositions peuvent contenir tous autres adjuvants habituellement utilisés en cosmétique et notamment dans le traitement des cheveux et/ou de la peau, tels que des parfums, des conservateurs, des sequestrants, des stabilisateurs de mousse, des agents propulseurs, des filtres solaires, des colorants, des agents acidifiants ou alcalinisants ou d'autres adjuvants selon l'usage envisagé.

Le procédé de lavage et/ou de conditionnement des matières kératiniques tel que les cheveux et/ou la peau, consiste à appliquer sur ces matières humides l'une quelconque des compositions définies ci-dessus et à procéder de préférence à un rinçage après leur application.

Les exemples suivants sont destinés à illustrer l'invention sans pour autant présenter un caractère limitatif.

EXEMPLE 1

On prépare un shampooing de composition suivante :

| | |
|---|---|
| - Tensio-actif de type acide éther carboxylique polyoxyéthyléné de formule : $R(OCH_2CH_2)_nOCH_2COOH$ dans laquelle : R = octylphényle n = valeur moyenne de 4 vendu sous la dénomination AKYPO OP 40 par la Société CHEM Y à 90% de MA | 5,0 g MA |
| - Laurylsulfate d'ammonium à 30% de MA | 5,0 g MA |
| - $\alpha$-oléfinesulfonate de sodium à 38% de MA | 5,0 g MA |
| - Polymère quaternisé vendu à la concentration de 60-63% de MA sous la dénomination MIRAPOL 175 par la Société MIRANOL | 1,0 g MA |
| - Polysiloxane à fonction hydroxyacylamino vendu sous la dénomination Q2-8413 par la Société DOW CORNING | 2,0 g |
| - Chlorure de sodium | 1,5 g |
| - Triéthanolamine qs pH=6,3 | |
| - Eau qsp | 100 g |

EXEMPLE 2

On prépare un shampooing de composition suivante :

| | |
|---|---|
| - Tensio-actif de type acide éther carboxylique polyoxyéthyléné de formule : $R(OCH_2CH_2)_nOCH_2COOH$ dans laquelle : R = octylphényle n = valeur moyenne de 4 vendu sous la dénomination AKYPO OP 40 par la Société CHEM Y à 90% de MA | 5,0 g MA |
| - Laurylsulfate d'ammonium à 30% de MA | 5,0 g MA |
| - $\alpha$-oléfinesulfonate de sodium à 38% de MA | 5,0 g MA |
| - Copolymère de chlorure de diméthyl diallyl ammonium et d'acrylamide vendu sous la dénomination MERQUAT 550 à 8% de MA par la Société MERCK | 0,5 g MA |
| - Polysiloxane à fonction hydroxyacylamino vendu sous la dénomination Q2-8413 par la Société DOW CORNING | 2,0 g |
| - Chlorure de sodium | 1,5 g |
| - Triéthanolamine qs pH=6,2 | |
| - Eau qsp | 100 g |

EXEMPLE 3

On prépare un shampooing de composition suivante :

| | |
|---|---|
| - Tensio-actif de type acide éther carboxylique polyoxyéthyléné de formule : $R(OCH_2CH_2)_nOCH_2COOH$ dans laquelle : R = octylphényle n = valeur moyenne de 4 vendu sous la dénomination AKYPO OP 40 par la Société CHEM Y à 90% de MA | 5,0 g MA |
| - Laurylsulfate d'ammonium à 30% de MA | 5,0 g MA |
| - $\alpha$-oléfinesulfonate de sodium à 38% de MA | 5,0 g MA |
| - Polymère P1 | 1,0 g |
| - Polysiloxane à fonction hydroxyacylamino vendu sous la dénomination Q2-8413 par la Société DOW CORNING | 2,0 g |
| - Chlorure de sodium | 1,5 g |
| - Triéthanolamine qs pH=6,3 | |
| - Eau qsp | 100 g |

EXEMPLE 4

On prépare un shampooing de composition suivante :

| | |
|---|---|
| - Tensio-actif de type acide éther carboxylique polyoxyéthyléné de formule : $R(OCH_2CH_2)_nOCH_2COOH$ dans laquelle : R = alkyle en $C_{12}$-$C_{14}$ n = valeur moyenne de 4,5 vendu sous la dénomination AKYPO RLM 45 par la Société CHEM Y à 90% de MA | 12,0 g MA |
| - Cocoamphocarboxyglycinate vendu à la concentration de 38% de MA sous la dénomination MIRANOL C2M conc. par la Société MIRANOL | 4,0 g MA |
| - Copolymère de chlorure de diméthyl diallyl ammonium et d'acrylamide, vendu sous la dénomination MERQUAT 550 à 8% de MA par la Société MERCK | 0,2 g MA |
| - Polysiloxane à fonction hydroxyacylamino vendu sous la dénomination Q2-8413 par la Société DOW CORNING | 4,0 g |
| - Dérivé de dioléate de polyéthylèneglycol (550E) et de propylèneglycol, vendu sous la dénomination ANTIL 141 Liquide par la Société GOLDSCHMIDT | 2,0 g MA |
| - Acide chlorhydrique qs pH=7,7 | |
| - Eau qsp | 100 g |

EXEMPLE 5

On prépare un shampooing de composition suivante :

| | |
|---|---|
| - Tensio-actif de type acide éther carboxylique polyoxyéthyléné de formule : $R(OCH_2CH_2)_nOCH_2COOH$ dans laquelle : R = alkyl($C_{12}$-$C_{14}$) n = valeur moyenne de 2,5 vendu sous la dénomination AKYPO RLM 25 par la Société CHEM Y à 90% de MA | 7,2 g MA |
| - Laurylsulfate d'ammonium à 30% de MA | 5,0 g MA |
| - $\alpha$-oléfinesulfonate de sodium à 38% de MA | 5,0 g MA |
| - Polymère quarternisé vendu à la concentration de 60-63% de MA sous la dénomination MIRAPOL 175 par la Société MIRANOL | 0,5 g MA |
| - Polysiloxane à fonction hydroxyacylamino vendu sous la dénomination Q2-8413 par la Société DOW CORNING | 10,0 g |
| - Dérivé de dioléate de polyéthylèneglycol (550E) et de propylèneglycol, vendu sous la dénomination ANTIL 141 Liquide par la Société GOLDSCHMIDT à 43,6% de MA | 1,5 g MA |
| - Diéthanolamide d'acide de coprah | 2,5 g |
| - Chlorure de sodium | 3,0 g |
| - Hydroxyde de sodium qs pH=5,6 | |
| - Parfum, conservateurs qs | |
| - Eau qsp | 100 g |

EXEMPLE 6

On prépare un gel-douche de composition suivante :

| | |
|---|---|
| - Lauryl($C_{12}$-$C_{14}$/70-30)sulfate de triéthanolamine vendu à 40% de MA | 30,0 g MA |
| - Hydroxyde d'alkyl($C_{12}$-$C_{18}$)diméthylcarboxyméthylammonium vendu à 32% de MA sous la dénomination DEHYTON AB 30 par la Société HENKEL | 3,2 g MA |
| - Polysiloxane à fonction hydroxyacylamino vendu sous la dénomination Q2-8413 par la Société DOW CORNING | 1,5 g |
| - Monoisopropanolamide de coprah | 2,5 g |
| - Distéarate d'éthylèneglycol($C_{16}$-$C_{18}$/70-30) | 2,0 g |
| - Polymère d'hydroxyéthylcellulose et d'épichlorhydrine quaternisé avec la triméthylamine, vendu sous la dénomination JR 400 par la Société UNION CARBIDE | 0,5 g |
| - Chlorure de sodium | 2,0 g |
| - Triéthanolamine qs pH=7 | |
| - Parfum, conservateurs qs | |
| - Eau qsp | 100 g |

**Revendications**

1. Composition de lavage et/ou de conditionnement des matières kératiniques, caractérisée par le fait qu'elle contient dans un milieu aqueux approprié pour ces matières, au moins une silicone à fonction hydroxyacylamino, au moins un polymère substantif et au moins un agent tensio-actif détergent.

2. Composition selon la revendication 1, caractérisée par le fait que la silicone à fonction hydroxylamino est choisie parmi les composés répondant à la formule :

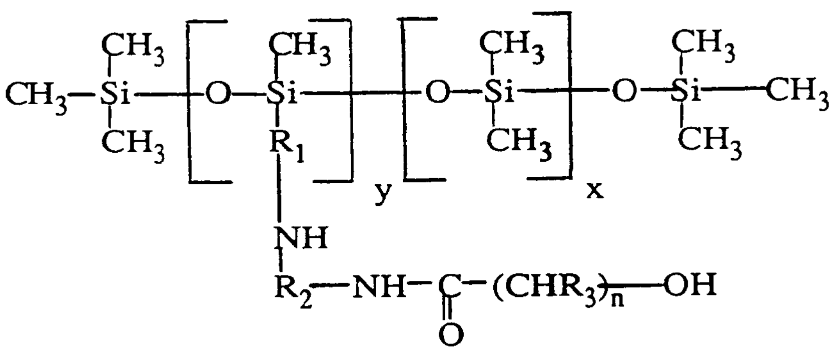

(I)

dans laquelle :
$R_1$ et $R_2$ désignent $(CH_2)_2$, $(CH_2)_3$, $(CH_2)_4$, $-CH_2CH(CH_3)CH_2-$ ;
$R_3$ désigne hydrogène, hydroxyle, alkyle, hydroxyalkyle;
n est un nombre entier variant de 2 à 7;
x est un nombre entier variant de 20 à environ 1500;
y varie entre environ 0,5 et 10;
et le rapport y/x est inférieur à 0,05.

3. Composition selon la revendication 1, caractérisée par le fait que la silicone à fonction hydroxyacylamino est une silicone répondant à la formule (I) :

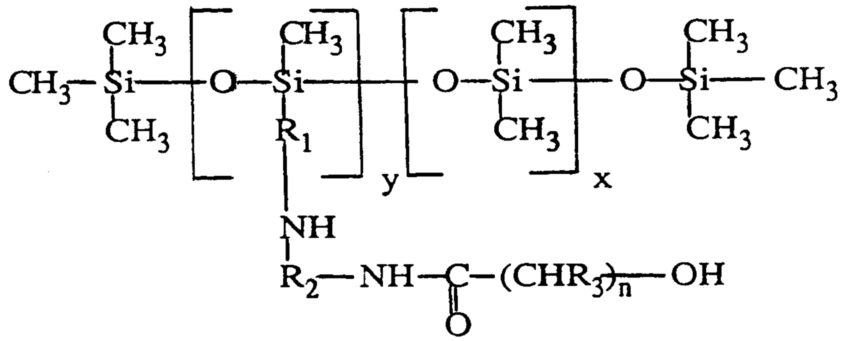

(I)

dans laquelle :
$R_1$ désigne $-CH_2CH(CH_3)CH_2-$;
$R_2$ désigne $-(CH_2)_2-$ ;
$R_3$ désigne hydrogène;
x est égal à 392;
y est égal à 8;
n est égal à 3.

**4.** Composition selon l'une quelconque des revendications 1 à 3, caractérisée par le fait que le polymère substantif est choisi parmi des polymères comportant des groupements amine primaire, secondaire, tertiaire et/ou quaternaire faisant partie de la chaîne polymère ou directement reliés à celle-ci et ayant un poids moléculaire compris entre 500 et environ 5.000.000 et de préférence entre 1000 et 3.000.000 et sont différents du copolymère de diallyl dialkyl ammonium ait d'un monomère anionique tel que l'acide acrylique.

**5.** Composition selon la revendication 4, caractérisée par le fait que le polymère substantif est choisi parmi les protéines quaternisées, les polymèrais de la famille des polyamines, polyaminoamides, polyammonium quaternaires ou des polymères dérivés du chitosane.

**6.** Composition selon la revendication 5, caractérisée par le fait que la protéine quaternisée est choisie parmi des polypeptides modifiés chimiquement et portant en bout de chaîne ou greffés sur celle-ci, des groupements ammonium quaternaires.

**7.** Composition selon la revendication 6, caractérisée par le fait que la protéine quaternisée est choisie parmi :

- les hydrolysats de collagène portant des groupements triéthylammonium;
- les hydrolysats de collagène portant des groupements chlorure de triméthylammonium ou de diméthylstéarylammonium;
- les hydrolysats de protéines animales portant des groupements diméthylbenzylammonium;
- les hydrolysats de protéines portant sur la chaîne polypeptidique des groupements ammonium quaternaires comportant un groupement alkyle ayant 1 à 18 atomes de carbone;
- des protéines quaternisées végétales de soja;
- les protéines quaternisées résultant de la condensation de la cocamidopropyldiméthylamine sur une protéine animale hydrolysée.

**8.** Composition selon la revendication, 5, caractérisée par le fait que les polymères de la famille des polyamines, polyaminoamides ou polyammonium quaternaires, sont choisis parmi :

(1) les copolymères vinylpyrrolidone/acrylate ou méthacrylate de dialkylaminoalkyle quaternisés ou non;
(2) les dérivés d'éthers de cellulose comportant des groupements ammonium quaternaires;

(3) les copolymères de cellulose ou de dérivés de cellulose greffés par un monomère hydrosoluble d'ammonium quaternaire;

(4) les polysaccharides quaternisés;

(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères;

(6) les polyaminoamides solubles dans l'eau qui sont des polycondensats d'un composé acide avec une polyamine, éventuellement réticulés par une épihalohydrine, un diépoxyde, un dianhydridrine, un anhydride non saturé, un dérivé bis-insaturé, une bis-halohydrine, un bis-azétidinium, une bis-haloacyldiamine, un bis-halogénure d'alkyle, ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé;

(7) les dérivés de polyaminoamides qui sont des produits de condensation de polyalkylène polyamines avec des acides polycarboxyliques alkylés par des agents bifonctionnels;

(8) les polymères qui sont des produits de réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés, ayant de 3 à 8 atomes de carbone, le polyaminoamide en résultant étant alkylé avec l'épichlorhydrine;

(9) les cyclopolymères ayant un poids moléculaire de 20 000 à 3 000 000 choisis parmi les homopolymères, comportant comme motif principal de la chaîne des motifs de formules (II) et (II'):

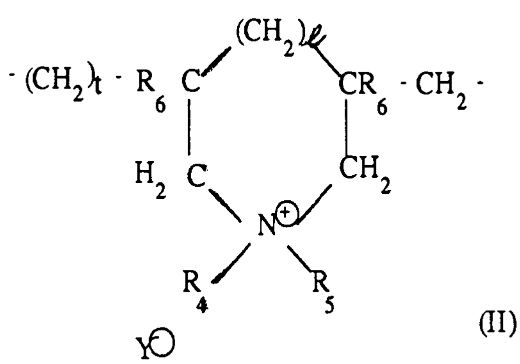
(II)

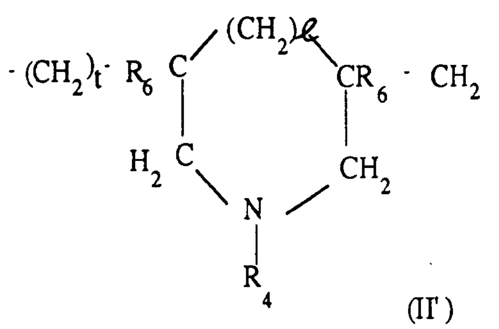
(II')

dans lesquelles :

$\ell$ et t sont égaux à 0 ou 1, et la somme $\ell + t = 1$ ;

$R_6$ désigne hydrogène ou méthyle;

$R_4$ et $R_5$ désignent, indépendamment l'un de l'autre, un groupement alkyle ayant de 1 à 22 atomes de carbone, un groupement hydroxyalkyle dans lequel le groupement alkyle a de préférence 1 à 5 atomes de carbone, un groupement amidoalkyle inférieur et où $R_4$ et $R_5$ peuvent désigner conjointement avec l'atome d'azote, auquel ils sont rattachés, des groupements hétérocycliques ainsi que les copolymères comportant des unités de formules (II) ou (II') et des unités dérivés d'acrylamide ou de diacétone acrylamide;

$Y^{\ominus}$ étant un anion;

(10) les polymères de polyammonium quaternaire;

(11) les homopolymères ou copolymères dérivés d'esters ou d'amides acrylique ou méthacrylique comportant les motifs :

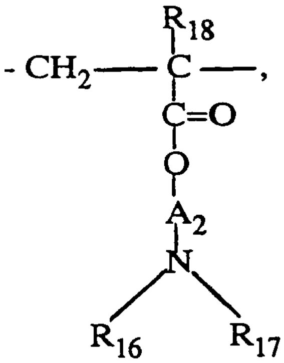
,
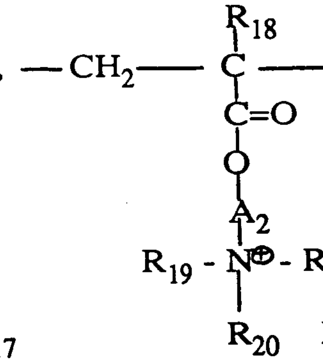
ou
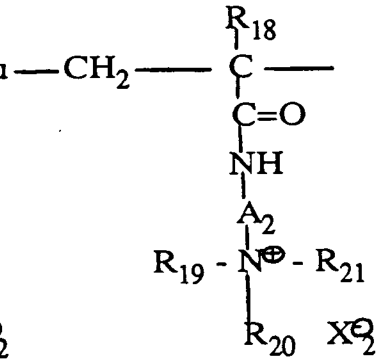
(V)

dans lesquels :

$R_{18}$ désigne H ou $CH_3$;

$A_2$ est un groupement alkyle linéaire ou ramifié de 1 à 6 atomes de carbone ou un groupe hydroxyalkyle de 1 à 4 atomes de carbone;

$R_{19}$, $R_{20}$, $R_{21}$, identiques ou différents, représentent un groupement alkyle ayant de 1 à 18 atomes de carbone ou un radical benzyle;

$R_{16}$ et $R_{17}$ représentent hydrogène ou un groupe alkyle ayant de 1 à 6 atomes de carbone;

$X^{\ominus}{}_2$ désigne un anion méthosulfate ou un halogénure;

(12) les polymères quaternaires de vinylpyrrolidone et de vinylimidazole;

(13) les polyamines, polyalkylèneimines, les polymères à motifs vinylpyridine ou vinylpiridinium, les condensats de polyamines et d'épichlorhydrine, les polyuréylènes quaternaires et les dérivés de la chitine.

**9.** Composition selon la revendication 5, caractérisée par le fait que les polymères dérivés de chitosane sont choisis parmi les polymères comportant les motifs (A), (B), (C):

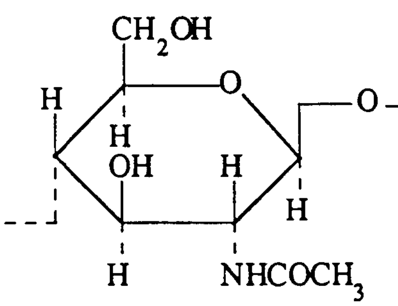
(A)

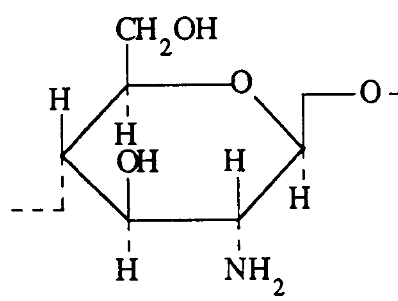
(B)

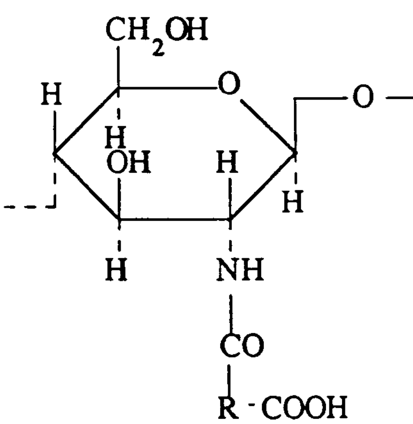
(C)

(VI)

dans laquelle le motif (A) est présent dans des proportions comprises entre 0 et 30%, le motif (B) est présent dans

des proportions de 5 à 50%, le motif (C) est présent dans des proportions de 30 à 90% en poids, R représente alkylène linéaire ou ramifié comportant de 2 à 5 atomes de carbone.

**10.** Composition selon l'une quelconque des revendications 1 à 9, caractérisée par le fait que les agents tensio-actifs détergents sont choisis parmi les agents tensio-actifs anioniques, amphotères, zwittérioniques, non-ioniques ou leurs mélanges.

**11.** Composition selon l'une quelconque des revendications 1 à 10, caractérisée par le fait que les silicones à fonction hydroxyacylamino sont présentes dans les compositions dans des proportions comprises entre 0,1 et 20% en poids par rapport au poids total de la composition et de préférence entre 0,5 et 10% en poids.

**12.** Composition selon l'une quelconque des revendications 1 à 11, caractérisée par le fait que les polymères substantifs sont présents dans les compositions dans des proportions comprises entre 0,05 et 8% en poids et de préférence entre 0,1 et 5% en poids par rapport au poids total de la composiiton.

**13.** Composition selon l'une quelconque des revendications 1 à 12, caractérisée par le fait que les agents tensio-actifs détergents sont présents dans les compositions dans des proportions comprises entre 5 et 50% en poids par rapport au poids total de la composition et en particulier entre 8 et 35% en poids.

**14.** Composition selon l'une quelconque des revendications 1 à 13, caractérisée par le fait qu'elle contient un agent de mise en suspension des silicones différent des alcools ayant 27 à 44 atomes de carbone et comportant un ou deux groupements éther et/ou thioéther ou sulfoxyde.

**15.** Composition selon la revendication 14, caractérisée par le fait que les agents de mise en suspension sont choisis parmi :

a) les composés de formule :

$$R_{22}X \qquad \text{(III)}$$

dans laquelle $R_{22}$ est un radical aliphatique à longue chaîne carbonée, éventuellement interrompue par des atomes d'oxygène, et X est un reste acide carboxylique, sulfurique ou phosphorique ou un radical dérivé d'un acide carboxylique ou d'un amide; ces composés de formule (III) sont choisis parmi ceux dans lesquels :

(i) $R_{22}$ est un radical alkyle ou alcényle en $C_{11}$-$C_{21}$ et X est :

. un groupement COOD où D est un radical mono- ou polyhydroxyalkyle dérivé d'un polyol en $C_2$-$C_3$ ou un radical $CH_2CH_2SO_3M$,

. un groupement $CO(OCH_2CH_2)_n$-OH où n a une valeur comprise entre 2 et 150,

. un groupement

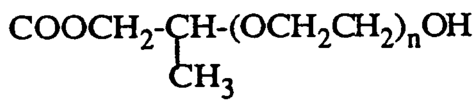

où n a une valeur comprise entre 2 et 150, les fonctions OH libres des groupements définis ci-dessus pouvant être estérifiées par un acide $R_{22}COOH$ où $R_{22}$ est un alkyle ou un alcényle en $C_{11}$-$C_{21}$,

. un groupement $CONR_{23}R_{24}$ où $R_{23}$ et $R_{24}$ représentent hydrogène ou hydroxyalkyle en $C_1$-$C_4$, l'un au moins représentant hydroxyalkyle en $C_1$-$C_4$,

. un groupement $OSO_3M$ ou 1/3 $PO_4^{3-}M_3$ où M représente un métal alcalin, ammonium ou un reste d'alconolamine en $C_1$-$C_4$;

(ii) $R_{22}$ désigne un radical $R_{25}(OC_2H_4)_\ell$ $OCH_2$ et X désigne un groupement COOM où M a la signification indiquée ci-dessus, $R_{25}$ désignant un radical alkyle en $C_{12}$-$C_{14}$ et $\ell$ un nombre entier ou décimal compris

entre 2,5 et 10, ou bien $R_{25}$ désigne oléyle et $\ell$ varie de 2 à 9 of encore $R_{25}$ désigne alkyle en ($C_8$-$C_9$)phényle et $\ell$ varie de 4 à 8, ou les dérivés dans lesquels $R_{22}$ désigne un groupement alkyle ($C_{12}$-$C_{16}$)éther et X un groupement $CONR_{23}R_{24}$, dans lequel $R_{23}$ et $R_{24}$ ont la même signification que celle indiquée ci-dessus;

b) des oxydes de diméthylalkyl($C_{16}$-$C_{22}$)amines;
c) les biopolysaccharides choisis par exemple parmi les gommes de xanthane et les scléroglucanes.

**16.** Composition selon la revendication 14 ou 15, caractérisée par le fait que les agents de mise en suspension des silicones sont présents dans des proportions comprises entre 0,1 et 20% en poids et de préférence entre 0,5 et 10% en poids par rapport au poids total de la composition.

**17.** Composition selon l'une quelconque des revendications 1 à 16, caractérisée par le fait que le milieu aqueux est constitué uniquement par de l'eau ou par un mélange d'eau et d'un solvant cosmétiquement acceptable.

**18.** Composition selon l'une quelconque des revendications 1 à 17, caractérisée par le fait que la composition contient en outre un adjuvant choisi parmi les agents nacrants ou opacifiants dans des proportions pouvant aller jusqu'à 3%, les agents régulateurs de viscosité dans des proportions pouvant aller jusqu'à 10% en poids par rapport au poids total de la composition.

**19.** Composition selon l'une quelconque des revendications 1 à 18, caractérisée par le fait que la composition contient en plus des agents tensio-actifs cationiques, des polymères autres que les polymères substantifs ou des protéines autres que les protéines à groupement ammonium quaternaire, des huiles, des cires, des résines ou gommes de silicone autres que les silicones à groupement hydroxyacylamino de formule (I).

**20.** Composition selon l'une quelconque des revendications 1 à 19, caractérisée par le fait que la composition contient des parfums, des conservateurs, des séquestrants, des stabilisateurs de mousse, des agents propulseurs, des colorants, des filtres solaires, des agents acidifiants ou alcalinisants ou tout autre adjuvant habituellement utilisé dans les compositions en cosmétique.

**21.** Procédé de lavage des matières kératiniques, caractérisé par le fait que l'on applique sur ces matières, au moins une composition telle que définie dans l'une quelconque des revendications 1 à 20 et qu'on procède après un certain temps de pose, à un rinçage à l'eau.

**22.** Procédé de conditionnement des cheveux, caractérisé par le fait que l'on applique sur ces cheveux, au moins une composition telle que définie dans l'une quelconque des revendications 1 à 20 et qu'on procède ensuite à leur rinçage.

**23.** Utilisation d'une composition telle que définie dans l'une quelconque des revendications 1 à 20, pour le lavage et/ou le conditionnement des matières kératiniques.

## Claims

**1.** Washing and/or conditioning composition for keratinous materials, characterised in that it contains, in an aqueous medium suitable for these materials, at least one silicone containing a hydroxyacylamino functional group, at least one substantive polymer and at least one detergent surface-active agent.

**2.** Composition according to Claim 1, characterised in that the silicone containing a hydroxyacylamino functional group is chosen from the compounds corresponding to the formula:

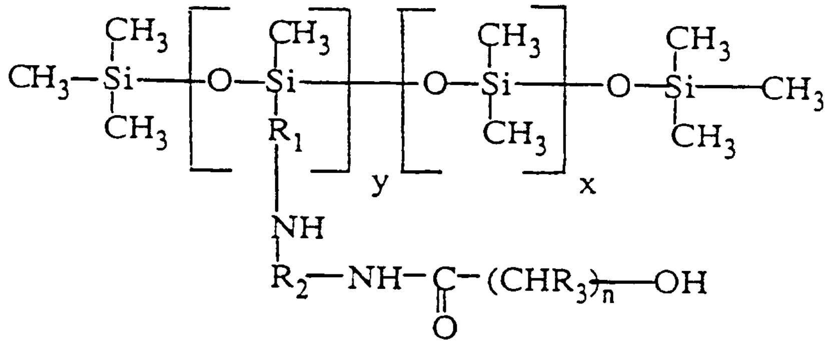

(I)

in which:
$R_1$ and $R_2$ denote $(CH_2)_2$, $(CH_2)_3$, $(CH_2)_4$ or $-CH_2CH(CH_3)CH_2-$;
$R_3$ denotes hydrogen, hydroxyl, alkyl or hydroxyalkyl;
n is an integer varying from 2 to 7;
x is an integer varying from 20 to approximately 1,500;
y varies between approximately 0.5 and 10;
and the y/x ratio is less than 0.05.

3. Composition according to Claim 1, characterised in that the silicone containing a hydroxyacylamino functional group is a silicone corresponding to the formula (I):

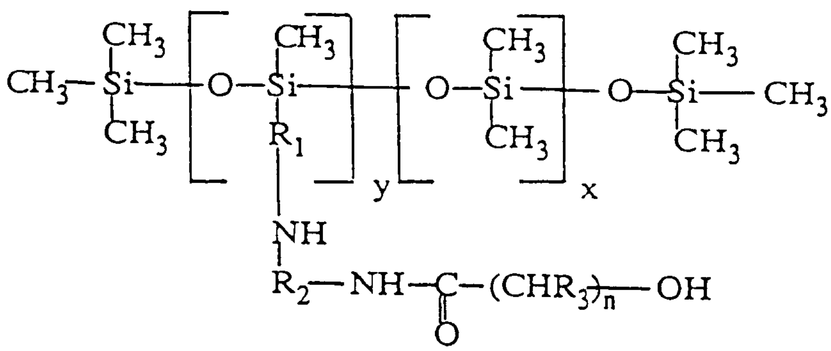

(I)

in which:
$R_1$ denotes $-CH_2CH(CH_3)CH_2-$;
$R_2$ denotes $-(CH_2)_2-$;
$R_3$ denotes hydrogen;
x is equal to 392;
y is equal to 8;
n is equal to 3.

4. Composition according to any one of Claims 1 to 3, characterised in that the substantive polymer is chosen from polymers containing primary, secondary, tertiary and/or quaternary amine groups which form part of the polymer chain or are directly connected to the latter and which have a molecular weight of between 500 and approximately 5,000,000 and preferably between 1,000 and 3,000,000 and are different from the copolymer of diallyldialkylammonium and of an anionic monomer such as acrylic acid.

5. Composition according to Claim 4, characterised in that the substantive polymer is chosen from the quaternised proteins, the polymers of the polyamine, polyaminoamide or quaternary polyammonium class or polymers derived from chitosan.

6. Composition according to Claim 5, characterised in that the quaternised protein is chosen from polypeptides which are chemically modified and which carry quaternary ammonium groups at the chain end or grafted on the latter.

**7.** Composition according to Claim 6, characterised in that the quaternised protein is chosen from:

- collagen hydrolysates carrying triethylammonium groups;
- collagen hydrolysates carrying trimethylammonium or dimethylstearylammonium chloride groups;
- animal protein hydrolysates carrying dimethylbenzylammonium groups;
- protein hydrolysates carrying, on the polypeptide chain, quaternary ammonium groups containing an alkyl group which has 1 to 18 carbon atoms;
- quaternised vegetable proteins from soya;
- quaternised proteins resulting from the condensation of cocamidopropyldimethylamine with a hydrolysed animal protein.

**8.** Composition according to Claim 5, characterised in that the polymers of the polyamine, polyaminoamide or quaternary polyammonium class are chosen from:

(1) optionally quaternised vinylpyrrolidone/dialkylaminoalkyl acrylate or methacrylate copolymers;
(2) cellulose ether derivatives containing quaternary ammonium groups;
(3) copolymers of cellulose or of cellulose derivatives grafted with a water-soluble quaternary ammonium monomer;
(4) quaternised polysaccharides;
(5) polymers consisting of piperazinyl units and of divalent, straight- or branched-chain alkylene or hydroxyalkylene radicals, optionally interrupted by oxygen, sulphur or nitrogen atoms or by aromatic or heterocyclic rings, as well as the oxidation and/or quaternisation products of these polymers;
(6) water-soluble polyaminoamides which are the polycondensates of an acid compound with a polyamine, optionally crosslinked by an epihalohydrin, a diepoxide, a dianhydride, an unsaturated anhydride, a bisunsaturated derivative, a bishalohydrin, a bisazetidinium, a bishaloacyldiamine, an alkyl bishalide or by an oligomer resulting from the reaction of a bifunctional compound reactive with respect to a bishalohydrin, a bisazetidinium, a bishaloacyldiamine, an alkyl bishalide, an epihalohydrin, a diepoxide or a bisunsaturated derivative;
(7) polyaminoamide derivatives which are condensation products of polyalkylenepolyamines with polycarboxylic acids alkylated by bifunctional agents;
(8) polymers which are reaction products of a polyalkylenepolyamine containing two primary amine groups and at least one secondary amine group with a dicarboxylic acid chosen from diglycolic acid and saturated aliphatic dicarboxylic acids having from 3 to 8 carbon atoms, the polyaminoamide resulting therefrom being alkylated with epichlorohydrin;
(9) cyclopolymers having a molecular weight of 20,000 to 3,000,000 chosen from the homopolymers containing, as the principal unit of the chain, units of formulae (II) and (II'):

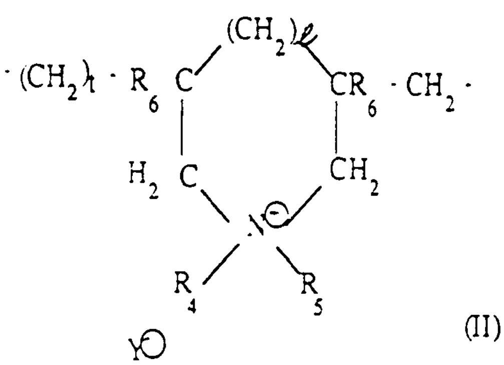
(II)

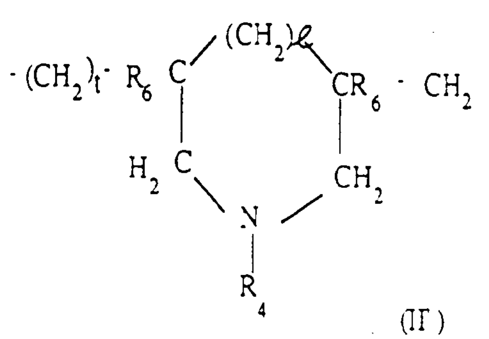
(II')

in which
$l$ and t are equal to 0 or 1, and the sum $l + t = 1$ ;
$R_6$ denotes hydrogen or methyl;
$R_4$ and $R_5$ denote, independently of each other, an alkyl group having from 1 to 22 carbon atoms, a hydroxyalkyl group in which the alkyl group preferably has 1 to 5 carbon atoms or a lower amidoalkyl group and where $R_4$ and $R_5$ can denote, jointly with the nitrogen atom to which they are connected, heterocyclic groups, as well as copolymers containing units of formulae (II) or (II') and units derived from acrylamide or from diacetoneacrylamide;
$Y^{\ominus}$ being an anion;
(10) quaternary polyammonium polymers;
(11) homopolymers or copolymers derived from acrylic or methacrylic esters or amides containing the units:

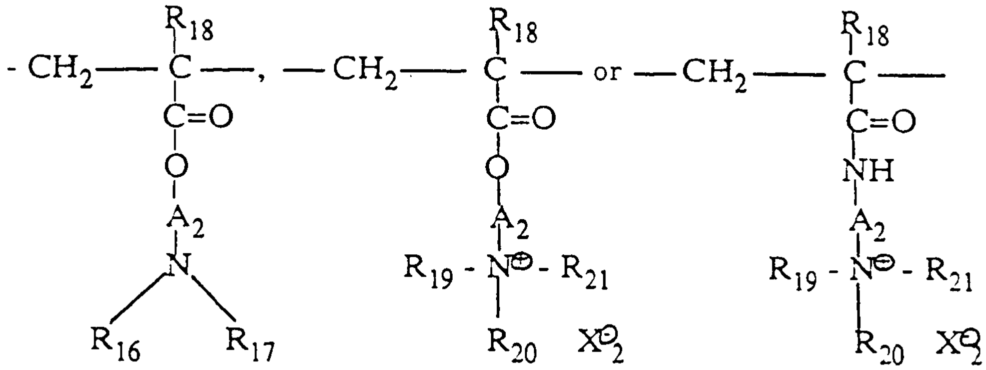

, or

(V)

in which:
$R_{18}$ denotes H or $CH_3$;
$A_2$ is a linear or branched alkyl group of 1 to 6 carbon atoms or a hydroxyalkyl group of 1 to 4 carbon atoms;
$R_{19}$, $R_{20}$ and $R_{21}$, which are identical or different, represent an alkyl group having from 1 to 18 carbon atoms or a benzyl radical;
$R_{16}$ and $R_{17}$ represent hydrogen or an alkyl group having from 1 to 6 carbon atoms;
$X^{\ominus}_2$ denotes a methosulphate anion or a halide;
(12) quaternary polymers of vinylpyrrolidone and of vinylimidazole;
(13) polyamines, polyalkyleneimines, polymers containing vinylpyridine or vinylpyridinium units, condensates of polyamines and of epichlorohydrin, quaternary polyureylenes and chitin derivatives.

9. Composition according to Claim 5, characterised in that the polymers derived from chitosan are chosen from the polymers containing the units (A), (B) or (C):

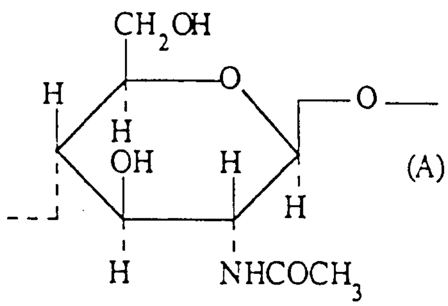

(A)

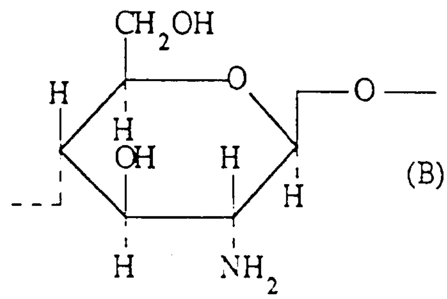

(B)

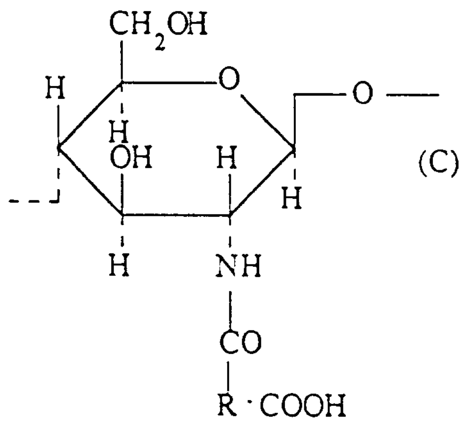

(C)

(VI)

in which unit (A) is present in proportions of between 0 and 30%, unit (B) is present in proportions of 5 to 50%, unit (C) is present in proportions of 30 to 90% by weight, R represents linear or branched alkylene containing from 2 to 5 carbon atoms.

**10.** Composition according to any one of Claims 1 to 9, characterised in that the detergent surface-active agents are chosen from the anionic, amphoteric, zwitterionic or nonionic surface-active agents or their mixtures.

**11.** Composition according to any one of Claims 1 to 10, characterised in that the silicones containing a hydroxyacylamino functional group are present in the compositions in proportions of between 0.1 and 20% by weight in relation to the total weight of the composition and preferably between 0.5 and 10% by weight.

**12.** Composition according to any one of Claims 1 to 11, characterised in that the substantive polymers are present in the compositions in proportions of between 0.05 and 8% by weight and preferably between 0.1 and 5% by weight in relation to the total weight of the composition.

**13.** Composition according to any one of Claims 1 to 12, characterised in that the detergent surface-active agents are present in the compositions in proportions of between 5 and 50% by weight in relation to the total weight of the composition and in particular between 8 and 35% by weight.

**14.** Composition according to any one of Claims 1 to 13, characterised in that it contains a suspending agent for the silicones other than alcohols having 27 to 44 carbon atoms and containing one or two ethers and/or thioether or sulphoxide groups.

**15.** Composition according to Claim 14, characterised in that the suspending agents are chosen from:

a) the compounds of formula:

$$R_{22}X \qquad \text{(III)}$$

in which $R_{22}$ is an aliphatic radical containing a long carbon chain, optionally interrupted by oxygen atoms, and X is a carboxylic, sulphuric or phosphoric acid residue or a radical derived from a carboxylic acid or from an amide; these compounds of formula (III) are chosen from those in which:

(i) $R_{22}$ is a $C_{11}$-$C_{21}$ alkyl or alkenyl radical and X is:

- a group COOD where D is a mono- or polyhydroxyalkyl radical derived from a $C_2$-$C_3$ polyol or a $CH_2CH_2SO_3M$ radical,
- a group $CO(OCH_2CH_2)_n$-OH where n has a value of between 2 and 150,
- a group

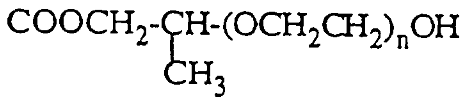

where n has a value of between 2 and 150, it being possible to esterify the free OH functional groups of the groups defined above with an acid $R_{22}COOH$ where $R_{22}$ is a $C_{11}$-$C_{21}$ alkyl or alkenyl,

- a group $CONR_{23}R_{24}$ where $R_{23}$ and $R_{24}$ represent hydrogen or $C_1$-$C_4$ hydroxyalkyl, one at least representing $C_1$-$C_4$ hydroxyalkyl,
- a group $OSO_3M$ or 1/3 $PO_4^{3-}M_3$ where M represents an alkali metal, ammonium or a $C_1$-$C_4$ alkanolamine residue;

(ii) $R_{22}$ denotes a radical $R_{25}(OC_2H_4)_\ell$ $OCH_2$ and X denotes a group COOM where M has the meaning shown above, $R_{25}$ denoting a $C_{12}$-$C_{14}$ alkyl radical and *1* an integer or decimal number of between 2.5 and 10, or else $R_{25}$ denotes oleyl and *1* varies from 2 to 9 or again $R_{25}$ denotes ($C_8$-$C_9$) alkylphenyl and *1* varies from 4 to 8, or the derivatives in which $R_{22}$ denotes a ($C_{12}$-$C_{16}$) alkyl ether group and X a group $CONR_{23}R_{24}$, in which $R_{23}$ and $R_{24}$ have the same meaning as that shown above;

b) dimethyl($C_{16}$-$C_{22}$) alkylamine oxides;
c) biopolysaccharides chosen, for example, from xanthan gums and scleroglucans.

**16.** Composition according to Claim 14 or 15, characterised in that the suspending agents for the silicones are present in proportions of between 0.1 and 20% by weight and preferably between 0.5 and 10% by weight in relation to the total weight of the composition.

**17.** Composition according to any one of Claims 1 to 16, characterised in that the aqueous medium consists solely of water or of a mixture of water and of a cosmetically acceptable solvent.

**18.** Composition according to any one of Claims 1 to 17, characterised in that the composition additionally contains an adjuvant chosen from pearlescence or opacifying agents in proportions which can range to up to 3%, and viscosity regulating agents in proportions which can range to up to 10% by weight in relation to the total weight of the composition.

**19.** Composition according to any one of Claims 1 to 18, characterised in that the composition contains, in addition to cationic surface-active agents, polymers other than substantive polymers or proteins other than proteins containing a quaternary ammonium group, oils, waxes, resins or silicone gums other than silicones containing a hydroxyacylamino group of formula (I).

**20.** Composition according to any one of Claims 1 to 19, characterised in that the composition contains fragrances, preservatives, sequestering agents, foam stabilisers, propellants, dyes, sunscreens, acidifying or alkalinising agents or any other adjuvant normally used in cosmetic compositions.

**21.** Process for washing keratinous materials, characterised in that at least one composition such as defined in any one of Claims 1 to 20 is applied to these materials and in that, after leaving for a certain period of time, a rinsing with water is carried out.

**22.** Process for conditioning hair, characterised in that at least one composition such as defined in any one of Claims 1 to 20 is applied to the hair and in that it is then rinsed.

23. Use of a composition such as defined in any one of Claims 1 to 20 for washing and/or conditioning keratinous materials.

**Patentansprüche**

1. Zusammensetzung zum Waschen und/oder Konditionieren keratinischer Materien, dadurch **gekennzeichnet**, daß sie in einem wässrigen, für diese Materien geeigneten Milieu mindestens ein Silicon mit Hydroxyacylamino-Funktion, mindestens ein substantives Polymer und mindestens ein oberflächenaktives reinigendes Mittel enthält.

2. Zusammensetzung gemäß Anspruch 1, dadurch **gekennzeichnet**, daß das Silicon mit Hydroxyacylamino-Funktion aus Verbindungen der Formel ausgewählt ist:

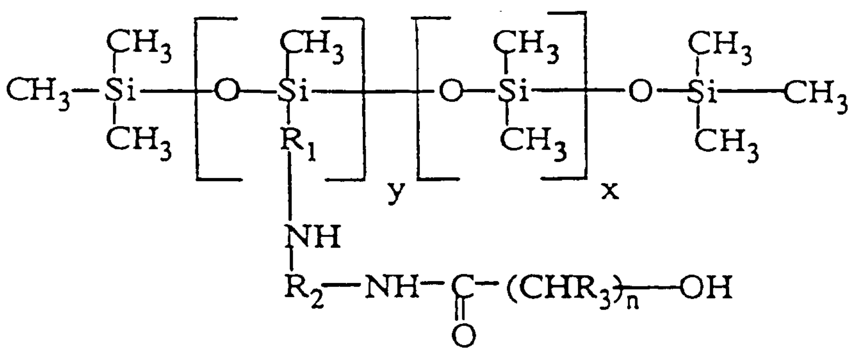

(I)

worin gilt:
$R_1$ und $R_2$ bedeuten $(CH_2)_2$, $(CH_2)_3$, $(CH_2)_4$, $-CH_2CH(CH_3)CH_2-$;
$R_3$ bedeutet Wasserstoff, einen Hydroxyl-, Alkyl- oder Hydroxyalkylrest;
n ist eine ganze Zahl von 2 bis 7;
x ist eine ganze Zahl von 20 bis ca. 1500;
y beträgt 0,5 bis 10;
und das Verhältnis y/x beträgt weniger als 0,05.

3. Zusammensetzung gemäß Anspruch 1, dadurch **gekennzeichnet**, daß das Silicon mit Hydroxyacylamino-Funktion ein Silicon der Formel (I) ist:

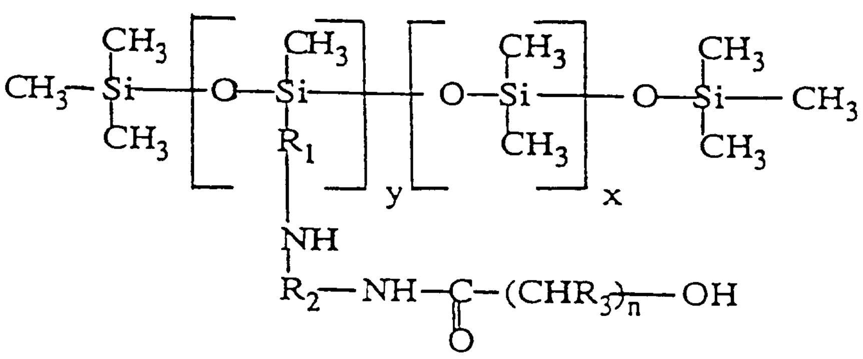

(I)

worin gilt:
$R_1$ bedeutet $-CH_2CH(CH_3)CH_2-$;
$R_2$ bedeutet $-(CH_2)_2-$;
$R_3$ bedeutet Wasserstoff;
x ist gleich 392;

y ist gleich 8;
n ist gleich 3.

4. Zusammensetzung gemäß einem der Ansprüche 1 bis 3, dadurch **gekennzeichnet**, daß das substantive Polymer aus Polieren ausgewählt ist, die primäre, sekundäre, tertiäre und/oder quaternäre Amingruppen aufweisen, die Teil der Polymerkette oder direkt an diese gebunden sind, ein Molekulargewicht von 500 bis ca. 5.000.000 und vorzugsweise von 1000 bis 3.000.000 haben und sich von Copolymeren aus Diallyldialkylammonium-Verbindungen und einem anionischen Monomer wie Acrylsäure unterscheiden.

5. Zusammensetzung gemäß Anspruch 4, dadurch **gekennzeichnet**, daß das substantive Polymer aus quaternierten Proteinen und Polymeren der Familie aus quaternären Polyaminen, Polyaminoamiden, Polyammonium-Polymeren oder Chitosan-Polymeren ausgewählt ist.

6. Zusammensetzung gemäß Anspruch 5, dadurch **gekennzeichnet**, daß das quaternierte Protein aus chemisch modifizierten Polypeptiden ausgewählt ist, die am Ende der Kette oder auf diese gepfropft quaternäre Anmoniumgruppen aufweisen.

7. Zusammensetzung gemäß Anspruch 6, dadurch **gekennzeichnet**, daß das quaternierte Protein ausgewählt ist aus:

- Kollagen-Hydrolysaten, die Triethylammoniumgruppen aufweisen;
- Kollagen-Hydrolysaten, die Trimethylammonium- oder Dimethylstearylammoniumchlorid-Gruppierungen aufweisen;
- Hydrolysaten tierischer Proteine, die Dimethylbenzylammoniumgruppen aufweisen;
- Protein-Hydrolysaten, die auf der Polypeptidkette quaternäre Ammoniumgruppen aufweisen, die eine Alkylgruppe mit 1 bis 18 Kohlenstoffatomen enthalten;
- quaternierten pflanzlichen Proteinen von Soja;
- quaternierten Proteinen aus der Kondensation von Cocoamidopropyldimethylamin an ein hydrolysiertes tierisches Protein.

8. Zusammensetzung gemäß Anspruch 5, dadurch **gekennzeichnet**, daß die Polymeren der Familie aus quaternären Polyaminen, Polyaminoamiden oder Polyammonium-Polymeren ausgewählt sind aus:

(1) gegebenenfalls quaternierten Copolymeren aus Vinylpyrrolidon/Dialkylaminoalkylacrylat oder -methacrylat;
(2) Derivaten von Celluloseethern, die quaternäre Ammoniumgruppen enthalten;
(3) Copolymeren von Cellulose oder Cellulosederivaten, die mit einem wasserlöslichen quaternären Ammonium-Monomer gepfropft sind;
(4) quaternierten Polysacchariden;
(5) Polymeren aus Piperazinyl-Einheiten und zweiwertigen Alkylen- oder Hydroxyalkylenresten mit geraden oder verzweigten Ketten, die gegebenenfalls durch Sauerstoff-, Schwefel-, Stickstoffatome oder durch aromatische oder heterozyklische Ringe unterbrochen sind, sowie aus Oxidations- und/oder Quaternierungsprodukten dieser Polymeren;
(6) wasserlöslichen Polyaminoamiden, die Polykondensate einer sauren Verbindung mit einem Polyamin sind, die gegebenenfalls mit einem Epihalohydrin, Diepoxid, Dianhydrid, ungesättigten Anhydrid, zweifach ungesättigten Derivat, Bishalohydrin, einer Bisazetidinium-Verbindung, einem Bishaloacyldiamin, Alkylbishalogenid oder auch mit einem Oligomer aus der Reaktion einer bifunktionellen Verbindung, die gegenüber einem Bishalohydrin, einer Bisazetidinium-Verbindung, einem Bishaloacyldiamin, Alkylbishalogenid, Epihalohydrin, Diepoxid oder einem zweifach ungesättigten Derivat reaktiv ist, vernetzt sind;
(7) Derivaten von Polyaminoamiden, die Kondensationsprodukte von Polyalkylenpolyaminen mit Polycarboxylsäuren sind, welche mit bifunktionellen Mitteln alkyliert sind;
(8) Polymeren, die Produkte aus der Reaktion eines Polyalkylenpolyamins, das zwei primäre Amingruppen und mindestens eine sekundäre Amingruppe aufweist, mit einer Dicarboxylsäure sind, die aus Diglycolsäure

und aliphatischen gesättigten Dicarboxylsäuren mit 3 bis 8 Kohlenstoffatomen ausgewählt ist, wobei das sich ergebende Polyaminoamid mit Epichlorhydrin alkyliert ist;

(9) Cyclopolymeren mit einem Molekulargewicht von 20000 bis 3.000.000, ausgewählt aus Homopolymeren, die als Grundeinheit der Kette Einheiten der Formeln (II) und (II') aufweisen:

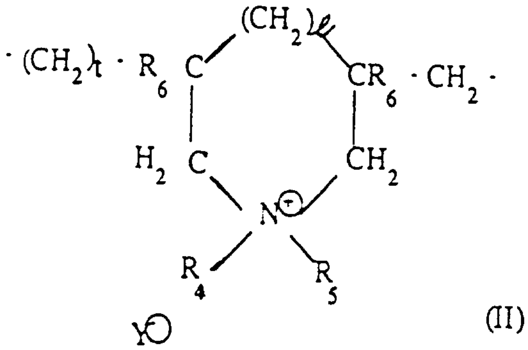
(II)

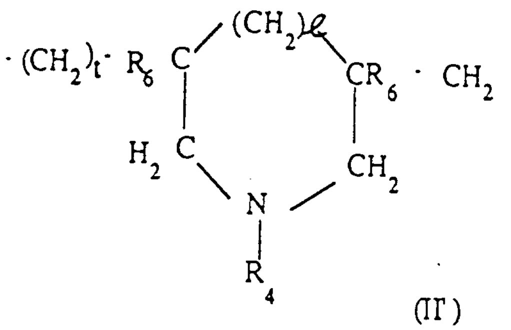
(II')

worin gilt:
l und t sind gleich 0 oder l, und die Summe l + t = 1 ;
$R_6$ bedeutet Wasserstoff oder einen Methylrest;
$R_4$ und $R_5$ bedeuten, unabhängig voneinander, eine Alkylgruppe mit 1 bis 22 Kohlenstoffatomen, eine Hydroxyalkylgruppe, in der die Alkylgruppe vorzugsweise 1 bis 5 Kohlenstoffatome aufweist, oder eine Aminoniedrigalkylgruppe, wobei $R_4$ und $R_5$ zusammen mit dem Stickstoffatom, an das sie gebunden sind, auch heterozyklische Gruppierungen bedeuten können, sowie aus Copolymeren aus Einheiten der Formeln (II) oder (II') und Einheiten, die von Acrylamid oder Diacetonamid abgeleitet sind;
$Y^-$ ist ein Anion;
(10) quaternären Polyammonium-Polymeren;
(11) Homopolymeren oder Copolymeren aus Acryl- oder Methacrylestern oder -amiden mit den Einheiten:

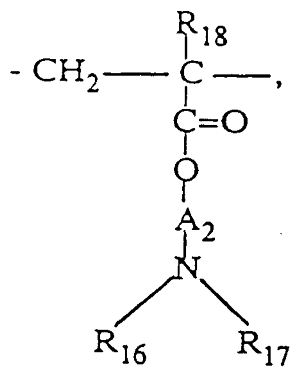
,
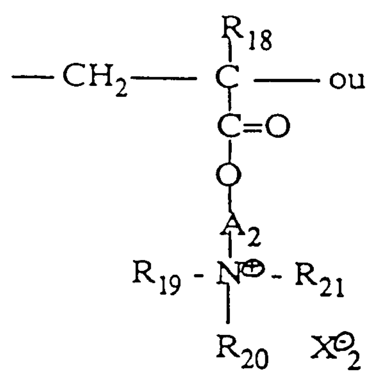
ou
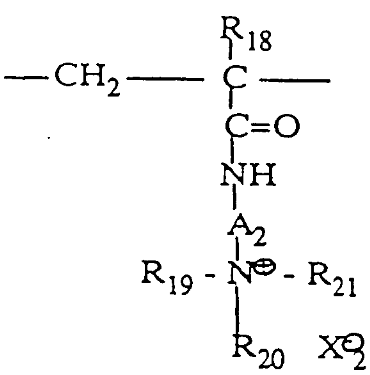
(V)

in denen gilt:
$R_{18}$ bedeutet H oder $CH_3$;
$A_2$ ist eine lineare oder verzweigte Alkylgruppe mit 1 bis 6 Kohlenstoffatomen oder eine Hydroxyalkylgruppe mit 1 bis 4 Kohlenstoffatomen, $R_{19}$, $R_{20}$, $R_{21}$ stellen, gleich oder verschieden, eine Alkylgruppe mit 1 bis 18 Kohlenstoffatomen oder einen Benzylrest dar;
$R_{16}$ und $R_{17}$ stellen Wasserstoff oder eine Alkylgruppe mit 1 bis 6 Kohlenstoffatomen dar;
$X^-_2$ bedeutet ein Methosulfat- oder Halogenid-Anion;
(12) quaternären Vinylpyrrolidon- und Vinylimidazol-Polymeren;
(13) Polyaminen, Polyalkyleniminen, Polymeren mit Vinylpyridin- oder Vinylpyridinium-Einheiten, Kondensaten aus Polyaminen und Epichlorhydrin, quaternären PolyharnstoffPolymeren und Chitin-Derivaten.

9. Zusammensetzung gemäß Anspruch 5,
dadurch **gekennzeichnet**, daß
die Chitosan-Polymeren aus Polymeren ausgewählt sind, die die Einheiten (A), (B) und (C) aufweisen:

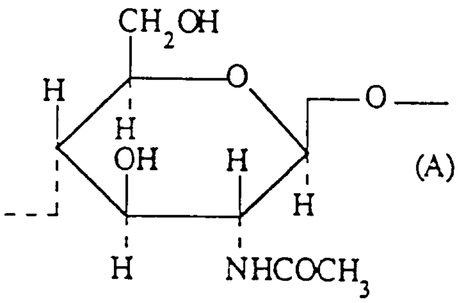

(A)

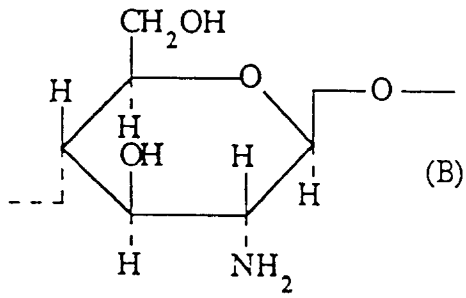

(B)

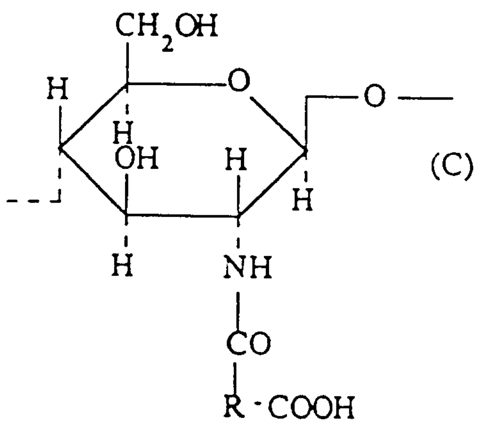

(C)

(VI)

worin die Einheit (A) in Mengenanteilen von 0 bis 30, die Einheit (B) in Anteilen von 5 bis 50 und die Einheit (C) in Anteilen von 30 bis 90 Gew.% vorhanden sind und R einen linearen oder verzweigten Alkylenrest mit 2 bis 5 Kohlenstoffatomen darstellt.

**10.** Zusammensetzung gemäß einem der Ansprüche 1 bis 9, dadurch **gekennzeichnet**, daß die oberflächenaktiven reinigenden Mittel aus oberflächenaktiven anionischen, amphoteren, zwitterionischen oder nicht-ionischen Mitteln oder aus deren Mischungen ausgewählt sind.

**11.** Zusammmensetzung gemäß einem der Ansprüche 1 bis 10, dadurch **gekennzeichnet**, daß die Silicone mit Hydroxyacylamino-Funktion in den Zusammensetzungen in Anteilen von 0,1 bis 20 und vorzugsweise von 0,5 bis 10 Gew.% vorliegen, bezogen auf das Gesamtgewicht der Zusammensetzung.

**12.** Zusammensetzung gemäß einem der Ansprüche 1 bis 11, dadurch **gekennzeichnet**, daß die substantiven Polymeren in den Zusammensetzungen in Anteilen von 0,05 bis 8 und vorzugsweise von 0,1 bis 5 Gew.% vorliegen, bezogen auf das Gesamtgewicht der Zusammensetzung.

**13.** Zusammensetzung gemäß einem der Ansprüche 1 bis 12, dadurch **gekennzeichnet**, daß die oberflächenaktiven Reinigungsmittel in den Zusammensetzungen in Anteilen von 5 bis 50 und insbesondere von 8 bis 35 Gew.% vorliegen, bezogen auf das Gesamtgewicht der Zusammensetzung.

**14.** Zusammensetzung gemäß einem der Ansprüche 1 bis 13, dadurch **gekennzeichnet**, daß sie ein Suspendiermittel für Silicone enthält, das sich von Alkoholen unterscheidet, die 27 bis 44 Kohlenstoffatome aufweisen und eine oder zwei Ether- und/oder Thioether- oder Sulfoxid-Gruppierungen enthalten.

**15.** Zusammensetzung gemäß Anspruch 14,
dadurch **gekennzeichnet**, daß
die Suspendiermittel ausgewählt sind aus:

a) Verbindungen der Formel:

$$R_{22}X \quad (III)$$

worin $R_{22}$ ein aliphatischer Rest mit langer Kohlenstoffkette, die gegebenenfalls mit Sauerstoffatomen unterbrochen ist, und X ein Carboxyl-, Schwefel- oder Phosphorsäurerest oder ein von einer Carboxylsäure oder einem Amid abgeleiteter Rest sind; diese Verbindungen der Formel (III) sind aus denjenigen ausgewählt, in denen gilt:

(i) $R_{22}$ ist ein $C_{11-21}$-Alkyl- oder -Alkenylrest, und X ist:

- eine Gruppierung COOD, worin D ein von einem $C_{2-3}$-Polyol abgeleiteter Mono- oder Polyhydroxyalkylrest oder ein $-CH_2CH_2SO_3M$-Rest ist,
- eine Gruppierung $CO(OCH_2CH_2)_n-OH$, worin n ein Wert von 2 bis 150 ist,
- eine Gruppierung $COOCH_2-C-CH_3-H-(OCH_2CH_2)_nOH$ worin n einen Wert von 2 bis 150 ist, wobei die von den oben definierten Gruppen freien OH-Funktionen mit einer Säure $R_{22}COOH$ verestert sein können, worin $R_{22}$ ein $C_{11-21}$-Alkyl-oder -Alkenylrest ist,
- eine Gruppierung $CONR_{23}R_{24}$, worin $R_{23}$ und $R_{24}$ ein Wasserstoffatom oder einen $C_{1-4}$-Hydroxyalkylrest darstellen, wobei mindestens einer einen $C_{1-4}$-Hydroxyalkylrest darstellt,
- eine Gruppierung $OSO_3M$ oder 1/3 $PO_4^{3-}M_3$, worin M ein Alkalimetall, einen Ammonium- oder $C_{1-4}$-Alkanolaminrest darstellt;

(ii) $R_{22}$ bedeutet einen Rest $R_{25}(OC_2H_4)_lOCH_2$, und X bedeutet eine Gruppierung COOM, worin M die oben angegebene Bedeutung hat, wobei $R_{25}$ einen $C_{12-14}$-Alkylrest und l eine ganze Zahl oder eine Dezimalzahl von 2,5 bis 10 oder $R_{25}$ auch einen Oleylrest und l 2 bis 9 oder $R_{25}$ auch einen $C_{8-9}$-Alkylphenylrest und l 4 bis 8 bedeuten, oder es sind auch Derivate eingeschlossen, in denen $R_{22}$ eine $C_{12-16}$-Alkylether-Gruppierung und X eine Gruppierung $CONR_{23}R_{24}$ bedeuten, worin $R_{23}$ und $R_{24}$ die oben angegebene Bedeutung haben;

b) Dimethyl-$C_{16-22}$-alkylaminoxiden;
c) Biopolysacchariden, ausgewählt z.B. aus Xanthan-Gummi und Scleroglucanen.

**16.** Zusammensetzung gemäß Anspruch 14 oder 15,
dadurch **gekennzeichnet**, daß
die Suspendiermittel der Silicone in Anteilen von 0,1 bis 20 und vorzugsweise von 0,5 bis 10 Gew.% vorliegen, bezogen auf das Gesamtgewicht der Zusammensetzung.

**17.** Zusammensetzung gemäß einem der Ansprüche 1 bis 16,
dadurch **gekennzeichnet**, daß
das wässrige Milieu nur aus Wasser oder aus einer Mischung aus Wasser und einem kosmetisch geeigneten Lösungsmittel zusammengesetzt ist.

**18.** Zusammensetzung gemäß einem der Ansprüche 1 bis 17,
dadurch **gekennzeichnet**, daß
die Zusammensetzung ausserdem einen Hilfsstoff enthält, der aus Perlmuttglanzmitteln oder opak machenden Mitteln in Mengenanteilen bis zu 3% und aus Viskositätsreguliermitteln in Anteilen bis zu 10 Gew.%, bezogen auf das Gesamtgewicht der Zusammensetzung, ausgewählt ist.

**19.** Zusammensetzung gemäß einem der Ansprüche 1 bis 18,
dadurch **gekennzeichnet**, daß
die Zusammensetzung zusätzlich oberflächenaktive kationische Mittel, Polymere, die sich von den substantiven Polymeren unterscheiden, oder Proteine, die sich von den Proteinen mit quaternären Ammoniumgruppen unterscheiden, Öle, Wachse, Harz- oder Gummiprodukte aus Silicon enthält, die sich von den Siliconen mit Hydroxyacylaminogruppe der Formel (I) unterscheiden.

**20.** Zusammensetzung gemäß einem der Ansprüche 1 bis 19,
dadurch **gekennzeichnet**, daß
die Zusammensetzung Parfüm-Produkte, Konservierungsstoffe, Sequestriermittel, Schaumstabilisatoren, Treibmittel, Farbstoffe, Sonnenfilterstoffe, sauer oder alkalisch stellende Mittel oder jeden weiteren, gewöhnlich in Zusammensetzungen für die Kosmetik verwendeten Hilfsstoff enthält.

**21.** Verfahren zum Waschen keratinischer Materien,
dadurch **gekennzeichnet**, daß
man auf diese Materien mindestens eine in einem der Ansprüche 1 bis 20 definierte Zusammensetzung aufbringt und nach einer bestimmten Verweilzeit mit Wasser spült.

**22.** Verfahren zum Konditionieren der Haare,
dadurch **gekennzeichnet**, daß
man auf die Haare mindestens eine in einem der Ansprüche 1 bis 20 definierte Zusammensetzung aufbringt und anschließend die Haare spült.

**23.** Verwendung einer in einem der Ansprüche 1 bis 20 definierten Zusammensetzung zum Waschen und/oder Konditionieren keratinischer Materien.